(19) Europäisches Patentamt
European Patent Office
Office européen des brevets

(11) **EP 2 710 426 B1**

(12) **EUROPÄISCHE PATENTSCHRIFT**

(45) Veröffentlichungstag und Bekanntmachung des Hinweises auf die Patenterteilung:
**16.12.2020  Patentblatt 2020/51**

(21) Anmeldenummer: **12723377.3**

(22) Anmeldetag: **16.05.2012**

(51) Int Cl.:
*G02B 3/02* (2006.01)     *G02B 27/00* (2006.01)
*G02C 7/06* (2006.01)     *A61F 2/16* (2006.01)

(86) Internationale Anmeldenummer:
**PCT/EP2012/002098**

(87) Internationale Veröffentlichungsnummer:
**WO 2012/156081 (22.11.2012 Gazette 2012/47)**

(54) **LINSE MIT EINEM ERWEITERTEN FOKUSBEREICH**

LENS WITH AN EXTENDED FOCAL RANGE

LENTILLE À PLAGE DE FOCALISATION AGRANDIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.05.2011  DE 102011101899**

(43) Veröffentlichungstag der Anmeldung:
**26.03.2014  Patentblatt 2014/13**

(73) Patentinhaber: **Carl Zeiss AG**
**73447 Oberkochen (DE)**

(72) Erfinder: **DOBSCHAL, Hans-Jürgen**
**99510 Kleinromstedt (DE)**

(74) Vertreter: **Rößner, Ulrike**
**Carl Zeiss AG**
**Patentabteilung Jena**
**Carl-Zeiss-Promenade 10**
**07745 Jena (DE)**

(56) Entgegenhaltungen:
EP-A1- 0 622 653      WO-A2-2008/087485
WO-A2-2008/087486     US-A1- 2003 117 577
US-A1- 2010 329 605

• **PRASAD SUDHAKAR ET AL: "Pupil-phase optimization for extended-focus, aberration-corrected imaging systems", PROCEEDINGS OF SPIE, SPIE, US, Bd. 5559, 1. Januar 2004 (2004-01-01), Seiten 335-345, XP002440110, ISSN: 0277-786X, DOI: 10.1117/12.560235**
• **SUDHAKAR PRASAD ET AL: "Engineering the pupil phase to improve image quality", PROCEEDINGS OF THE SPIE, SPIE, US, Bd. 5108, 21. April 2003 (2003-04-21), Seiten 1-12, XP002632539, ISSN: 0277-786X, DOI: 10.1117/12.487572 [gefunden am 2004-02-03]**

**Beschreibung**

Die Erfindung betrifft eine Linse, welche einen erweiterten Fokusbereich aufweist, wobei die Linse aus einem festen Material besteht, die optischen Flächen der Linse transparent sind und die Linse eine Brechkraftverteilung aufweist. Weiterhin betrifft die Erfindung ein Verfahren zur Herstellung der Linse sowie ein Objektiv mit einem erweiterten Fokusbereich.

Multifokale Linsen sollen gleichzeitig mehrere Forderungen erfüllen. Zunächst soll eine hinreichend gute Kontrastübertragungsfunktion in zwei oder mehr Fokusebenen gewährleistet werden. Weiterhin soll die Kontrastübertragungsfunktion unabhängig von der Größe der Pupille sein. Und schließlich soll die Linse leicht herstellbar sein, sie soll keine Absätze sowie Kanten und daher möglichst glatte Kurven aufweisen.

Derartige Linsen werden insbesondere bei der Korrektur von Sehfehlern mittels Brillengläsern oder als Intraokularlinsen (IOL-Linsen) verwendet.

Im Gegensatz zu den schon seit vielen Jahren eingeführten monofokalen IOL-Linsen sind multifokale Linsen bisher nur für den bifokalen Fall umgesetzt, da es erhebliche Probleme bereitet, die oben genannten Forderungen gleichzeitig zu erfüllen. Eine Variante basiert hierbei auf einem speziellen rotationssymmetrischen Ringsystem, wobei durch eine geschickte Abstimmung von Ringradien, Ringbreiten und Ringtiefen eine ausreichend gute Abbildung für zwei diskrete objektseitige Brennebenen erfolgt, beispielsweise bei 0 dpt und ca. 3 dpt Korrekturstärke.

Eine derartige bifokale Linse wird in der Patentschrift US 5982543 A beschrieben und verwendet ein rotationssymmetrisches Fresnel-ähnliches Ringsystem.

In US 6120148A wird ein rotationssymmetrisches diffraktives Ringsystem beschrieben. Die bifokale Linse aus US 6536899 B verwendet ebenfalls ein Ringsystem, wobei jeder Ring aus zwei Subringen besteht, die jeweils die zwei gewünschten Brennweiten realisieren.

In etwas modifizierter Form sind hieraus auch Lösungen abgeleitet, bei denen eine einzige Linse einen erweiterten, kontinuierlichen Fokusbereich abdeckt. Derartige Linsen sind auch unter dem Begriff "Extended Depth of Focus-Linse" oder auch "EDoF-Linse" bekannt. In US 2006176572 A wird ein rotationssymmetrisches System aus Ringen verwendet, wobei die Einzelbrennweiten der Ringe innerhalb des gewünschten kontinuierlichen Brennweitenbereiches liegen. Der "Extended Depth of Focus"-Effekt entsteht durch die Vermischung der verschieden Brennweiten.

Das System gemäß WO 100 835 46A besteht aus Sektoren ("Tortenstücken") mit azimutal ansteigender Brechkraft. Die Brechkraftverteilung weist hierbei diskrete Sprünge zwischen den Sektoren auf. In EP 0 622 653 A1 ist eine Linse mit einer spiralförmigen Struktur offenbart, die eine Vielzahl von Brechkräften bereitstellt.

In US20100002310 A1 ist ein optisches Abbildungssystem für eine Kamera beschrieben, welches einen erweiterten Schäftentiefenbereich aufweist. Die erweiterte Schärfentiefe wird durch eine Kombination mehrerer Linsen mit asphärischen Oberflächen erreicht.

Die Aufgabe der Erfindung besteht darin, eine neue Linse mit einem erweiterten Fokusbereich zu schaffen. Die neue Linse soll einzeln, insbesondere als Intraokularlinse oder in Verbindung mit anderen optischen Komponenten optische Systeme liefern, welche bei einer hinreichend guten Abbildungsqualität einen großen Schärfentiefenbereich liefern und kostengünstig herstellbar sind.

Die Aufgabe der Erfindung wird erfindungsgemäß für die neue Linse mit den Merkmalen des Anspruchs 1 gelöst. Die Aufgabe der Erfindung wird erfindungsgemäß für die neue Linse mit den Merkmalen des Anspruchs 2 gelöst. Die Aufgabe der Erfindung wird erfindungsgemäß bei dem Verfahren zur Herstellung der Linse mit den Merkmalen des Anspruchs 11 gelöst. Vorteilhafte Weiterbildungen der Erfindung sind Gegenstand der Unteransprüche. Die Aufgabe der Erfindung wird erfindungsgemäß bei einem Objektiv mit einem erweiterten Fokusbereich mit den Merkmalen des Anspruchs 15 gelöst.

Die Erfindung betrifft eine Linse mit einem erweiterten Fokusbereich, wobei die Linse aus einem festen, transparenten Material besteht sowie zwei gefertigte optische Flächen hat. Die Linse hat eine Brechkraftverteilung $F_G$, die sich bezogen auf eine zur optischen Achse senkrecht stehende Ebene als Funktion der radialen Höhe r und des Azimutwinkels phi der Apertur zwischen einem Grundwert der Brechkraft $F_L$ ungleich Null und einem Maximalwert $F_{Smax}$ ungleich Null ändert.

Somit ergibt sich die Brechkraftverteilung zu $F_G(r, phi) = F_L + F_S(r, phi)$, wobei $F_S(r,phi) = F_{Smax}(r) * w(phi)$ ein spiralförmiger Brechkraftanteil ist. In der Formel $F_S(r,phi) = F_{Smax}(r) * w(phi)$ ist $F_{Smax}(r)$ nichtlinear radienabhängig und w(phi) ist ein Faktor für den Brechkraftanteil mit dem spiralförmigen Verlauf, welcher allgemein mit der Formel

$$w(phi) = \sum_{i=1}^{M} I_i \exp\left[-a_i(phi - w_i)^2\right]$$

beschrieben ist, wobei $w_i$ die Peaklagen der Winkelverteilungsfunktion; $I_i$ Intensitätswerte der Einzelpeaks mit $I_i > 0$; $a_i > 0$ Dämpfungskoeffizienten für die jeweiligen Peaklagen sowie i ein Zählwert und $M \geq i$ ein Endwert sind.

Die Erfindung betrifft weiterhin eine Linse mit einem erweiterten Fokusbereich, wobei die Linse aus einem festen, transparenten Material besteht sowie zwei gefertigte optische Flächen hat. Die Linse hat eine Brechkraftverteilung $F_G$, die sich bezogen auf eine zur optischen Achse senkrecht stehende Ebene als Funktion der radialen Höhe r und des Azimutwinkels phi der Apertur zwischen einem Grundwert der Brechkraft $F_L$ ungleich Null und einem Maximalwert $F_{Smax}$ ungleich Null ändert. Somit ergibt sich die Brechkraftverteilung zu $F_G(r,phi) = F_L + F_S(r,phi)$, wobei $F_S(r,phi) = F_{Smax}(r) * w(phi)$ ein spiralförmiger Brechkraftanteil ist.

In der Formel $F_S(r,phi) = F_{Smax}(r) * w(phi)$ ist $F_{Smax}(r)$ nichtlinear radienabhängig, und w(phi) ist ein Faktor für den Brechkraftanteil mit dem spiralförmigen Verlauf, der mit der Formel $$w(phi) = \frac{phi}{2\pi}$$ als linearer Verlauf beschrieben ist für Azimutwinkel phi von Null bis $2\pi$.

Die Linse zeichnet sich dadurch aus, dass die maximale Brechkraft $F_{Smax}(r)$ nichtlinear radienabhängig ist und

$$F_{S\max}(r) = \sum_{j=2}^{N} c_j * r^j \qquad F_{S\max}(r) = \sum_{j=1}^{N} c_j * r^{2*j}$$

mit den Polynomformeln                              oder                                        mit den Polynomkoeffizi-

$$F_{S\max}(r) = \sum_{j=2}^{N} k_j * r^j \qquad F_{S\max}(r) = \sum_{j=1}^{N} k_j * r^{2*j}$$

enten $c_j$ für eine refraktive Brechkraft und          oder                                        mit dem Polynomkoeffizienten $k_j$ für eine diffraktive Brechkraft beschrieben ist, wobei j ein Zählwert und N≥j ein Endwert sind.

Um weitere Freiheitsgrade zur Dimensionierung der Linse zu erhalten ist die maximale Brechkraft $F_{Smax}$ nichtlinear radienabhängig und zusätzlich vom Azimutwinkel phi der Apertur abhängig. Im diesem Fall gelten die Polynom-

$$F_{S\max}(r,phi) = \sum_{j=2}^{N} c_j(phi) * r^j \qquad F_{S\max}(r,phi) = \sum_{j=1}^{N} c_j(phi) * r^{2*j}$$

formeln                                          oder                                        mit den Polynomkoeffizi-

$$F_{S\max}(r,phi) = \sum_{j=2}^{N} k_j(phi) * r^j$$

enten    $c_j$    für    eine    refraktive    Brechkraft    und                                                        oder

$$F_{S\max}(r,phi) = \sum_{j=1}^{N} k_j(phi) * r^{2*j}$$

mit dem Polynomkoeffizienten $k_j$ für eine diffraktive Brechkraft, wobei j ein Zählwert und N≥j ein Endwert sind.

In einem ersten Fall ergibt sich die Brechkraftverteilung $F_G(r,phi)$ der Linse aus einem Höhenprofil $z_G(r, phi)$ einer zu fertigenden zweiten optischen Fläche. Die Form der zu fertigenden Fläche ergibt sich aus der Addition des Höhenprofils $z_L(r)$ einer berechneten Basisfläche und eines Höhenprofils $z(r, phi)$, wobei gilt

$$F_G(r,phi) = F_L + F_S(r,phi) => z_G(r,phi) = z_L(r) + z(r,phi)$$

Die additive Höhe $z(r, phi)$ ändert sich ausgehend von Null bis zu einem Maximalwert $z_{max}(r)$, welcher die maximale Brechkraft $F_{Smax}(r)$ liefert, nichtlinear radienabhängig und ergibt sich als Funktion

$$z(r,phi) = z_{\max}(r) * w(phi)$$

mit

$$z_{\max}(r) = \sum_{j=2}^{N} c_j * r^j \qquad z_{\max}(r) = \sum_{j=1}^{N} c_j * r^{2*j} ,$$

oder                                        wobei der Radius r zwischen 0 und D/2 und der Azimutwinkel phi der Apertur zwischen 0 und $2\pi$ sich stetig ändern, wodurch die zu fertigende optische Fläche mit dem spiralförmigen Höhenprofil beschrieben ist.

In einer alternativen Variante ändert sich die additive Höhe $z(r, phi)$ ausgehend von Null bis zu einem Maxi-

malwert $z_{max}$(r,phi), welcher die maximale Brechkraft $F_{Smax}$(r,phi) liefert, nichtlinear radienabhängig und vom Azimutwinkel phi der Apertur abhängig und ergibt sich als Funktion

$$z(r, phi) = z_{\max}(r, phi) * w(phi)$$

mit

$$z_{\max}(r, phi) = \sum_{j=2}^{N} c_j(phi) * r^j \qquad z_{\max}(r, phi) = \sum_{j=1}^{N} c_j(phi) * r^{2*j}$$

oder

.

In einem zweiten Fall ergibt sich der Brechkraftanteil mit dem spiralförmigen Verlauf $F_S$(r,phi) aus der Wirkung eines optischen Gitters, welches auf eine gefertigte zweite optische Fläche mit der Brechkraft $F_L$ aufgebracht ist, wobei gilt $F_G$(r, phi) = $F_L$ + $F_S$(r, phi).

Die Frequenz des optischen Gitters ändert sich von einem Grundwert Null aus bis zu einem Maximalwert $Phase_{max}$, welche die maximale Brechkraft $F_{Smax}$ liefert, nichtlinear radienabhängig. Für den spiralförmigen Brechkraft-

$$Phase_{\max}(r) = \sum_{j=2}^{N} k_j * r^j$$

verlauf gilt $F_S$(r,phi) = $F_{S\,max}$(r) * w(phi) => $Phase$(r,phi) = $Phase_{max}$(r) * w(phi) mit     oder

$$Phase_{\max}(r) = \sum_{j=1}^{N} k_j * r^{2*j} \, ,$$

wobei der Radius r zwischen 0 und D/2 und der Azimutwinkel phi der Apertur zwischen 0 und $2\pi$ sich stetig ändern, wodurch das optische Gitter einen spiralförmigen Phasenverlauf aufweist.

In einer alternativen Variante ändert sich die Frequenz des optischen Gitters von einem Grundwert Null ausgehend bis zu einem Maximalwert $Phase_{max}$, welche die maximale Brechkraft $F_{Smax}$(r,phi) liefert, nichtlinear radienabhängig und ist vom Azimutwinkel phi der Apertur abhängig. Für den spiralförmigen Brechkraftverlauf gilt

$$F_S(r, phi) = F_{S\,max}(r, phi) * w(phi) =>$$

$$Phase(r, phi) = Phase_{max}(r, phi) * w(phi)$$

$$Phase_{\max}(r, phi) = \sum_{j=2}^{N} k_j(phi) * r^j \qquad Phase_{\max}(r, phi) = \sum_{j=1}^{N} k_j(phi) * r^{2*j} \, .$$

mit     oder

In einem dritten Fall ergibt sich der Brechkraftanteil mit dem spiralförmigen Verlauf Fs durch eine additive oder subtraktive Brechungsindexverteilung $\Delta n$(r,phi), wobei das Material der Linse eine Brechungsindexverteilung aufweist, die sich von einem Grundwert $n_2$ ausgehend bis zu einem Maximalwert $\Delta n_{max}$ nichtlinear radienabhängig ändert, wobei gilt $F_G$(r, phi) = $F_L$ + $F_S$(r, phi), und für den spiralförmigen Brechkraftverlauf gilt $F_S$(r,phi) = $F_{S\,max}$(r) * w(phi) => $\Delta n$(r, phi) =

$$\Delta n_{\max}(r) = \sum_{j=2}^{N} c_j * r^j \qquad \Delta n_{\max}(r) = \sum_{j=1}^{N} c_j * r^{2*j} \, ,$$

$\Delta n_{max}$(r) * w(phi) mit     oder     wobei der Radius r zwischen 0 und D/2 und der Azimutwinkel phi der Apertur zwischen 0 und $2\pi$ sich stetig ändern, wodurch eine spiralförmige Brechungsindexverteilung des Linsenmaterials beschrieben ist.

In einer alternativen Variante weist das Material der Linse (1) eine Brechungsindexverteilung auf, die sich von einem Grundwert $n_2$ ausgehend bis zu einem Maximalwert $\Delta n_{max}$ nichtlinear radienabhängig und vom Azimutwinkel phi der Apertur abhängig ändert, wobei gilt

$$F_G(r, phi) = F_L + F_S(r, phi) ,$$

und für den spiralförmigen Brechkraftverlauf gilt

$$\Delta n_{\max}(r, phi) = \sum_{j=2}^{N} c_j(phi) * r^j$$

$F_S(r, phi) = F_{S\,max}(r, phi) * w(phi) => \Delta n(r, phi) = \Delta n_{max}(r, phi) * w(phi)$ mit

$$\Delta n_{\max}(r, phi) = \sum_{j=1}^{N} c_j(phi) * r^{2*j} \, .$$

oder

Die beschriebenen Varianten liefern spezielle neue Linsenformen, mit denen es möglich ist, einen vorgegebenen Brennweitenbereich simultan abzudecken, das heißt über einen ausgedehnten Fokalbereich eine hinreichend gute Bildqualität zu generieren.

Derartige Linsen mit einem erweiterten Fokusbereich finden in optischen Systemen für eine Kamera, ein Mikroskop oder optischen Messeinrichtungen Anwendung.

Ein Hauptanwendungsgebiet ist eine Intraokularlinse mit variablem Brennweitenbereich, die einen Fokussierbereich von 0 bis ca. 3.5 dpt bezogen auf eine feste Basisbrechkraft realisiert. Eine solche Intraokularlinse wird meist nach Entfernung der natürlichen Linse ins Auge implantiert. Diese kann aber auch zusätzlich zur natürlichen Linse eingesetzt werden. Es ist auch vorgesehen, eine spiralförmige Brechkraftverteilung in die natürliche Augenlinse aufzuprägen oder einzuarbeiten.

Die erfindungsgemäße Linse wird in folgenden Schritten hergestellt:

Schritt 1: Berechnung eines monofokalen Basissystems mit einer Grundbrechkraft $F_L$ unter Festlegung der Parameter einer ersten optischen Fläche, der Parameter einer optischen Basisfläche und einer Linsendicke d sowie einer Werkstoffart mit einem Brechungsindex und einer Abbe-Zahl,

Schritt 2: Aufaddieren oder subtrahieren einer zusätzlichen Brechkraftverteilung $F_S(r,phi)$, welche sich bezogen auf eine zur optischen Achse senkrecht stehende Ebene als Funktion der radialen Höhe r und des Azimutwinkels phi der Apertur zwischen einem Grundwert und einem Maximalwert $F_{Smax}(r)$ oder $F_{Smax}(r,phi)$ nichtlinear radienabhängig ändert, womit die zusätzliche Brechkraft $F_S(r,phi)$ spiralförmig veränderlich über die Basisfläche verteilt wird.

Schritt 3: Herstellung der spiralförmigen Brechkraftverteilung an der Linse und/oder auf der Linse und/oder innerhalb der Linse.

Das "Aufaddieren" der spiralförmigen Brechkraftverteilung kann durch mehrere Varianten erfolgen, welche jeweils einzeln oder in beliebiger Kombination miteinander eingesetzt werden:

a) "Aufaddieren" eines spiralförmigen Höhenprofils z(r, phi) auf eine der im Schritt 1 berechneten Flächen der Linse, welche die Basisfläche ist, wobei damit das Profil der zweiten optischen Fläche festgelegt ist.
Die erfindungsgemäße Linse wird durch ein Verfahren hergestellt, bei dem die zusätzliche Brechkraftverteilung $F_S(r,phi)$, welche sich zwischen dem Grundwert und dem Maximalwert ändert, durch eine Addition eines spiralförmigen Höhenprofils z(r,phi) auf die berechnete Basisfläche des Basissystems erzeugt wird, wobei die additive Höhe z eine Funktion des Radius r und des Azimutwinkels phi der Apertur ist. Dabei ändert sich die additive Höhe z zwischen dem Wert Null und einem Maximalwert $z_{max}(r)$ oder $z_{max}(r,phi)$ nichtlinear radienabhängig. Somit liegt ein spiralförmiges Höhenprofil der zu fertigenden zweiten optischen Fläche fest.
b) "Aufaddieren" einer spiralförmigen diffraktiven Struktur auf eine der berechneten Flächen der Linse gemäß Schritt 1.
Hier wird die zusätzliche Brechkraftverteilung $F_S(r,phi)$, welche sich zwischen dem Grundwert und dem Maximalwert ändert, durch eine Addition der Wirkung eines spiralförmigen Gitterprofils Phase (r,phi) auf der gefertigten zweiten optische Fläche erzeugt. Dabei entspricht die gefertigte zweite optische Fläche der berechneten Basisfläche und das Gitterprofil ist eine Funktion des Radius r und des Azimutwinkels phi der Apertur. Die Brechungswirkung ändert sich zwischen dem Wert Null und dem Maximalwert $Phase_{max}(r)$ oder $Phase_{max}(r,phi)$ nichtlinear radienabhängig. Somit wird das spiralförmige optische Gitter auf der gefertigten optischen Fläche aufgebracht.
c) "Aufaddieren" eines spiralförmigen Brechungsindexverlaufes im Material der Linse. In diesem Fall werden die berechneten Flächen gemäß Schritt 1 nicht verändert. Die zweite optische Fläche entspricht der Basisfläche.
Die Brechkraftverteilung $F_S(r,phi)$, welche sich zwischen dem Grundwert und dem Maximalwert $\Delta n_{max}(r)$ oder $\Delta n_{max}(r,phi)$ nichtlinear radienabhängig ändert, wird durch einen spiralförmigen Brechungsindexverlauf $\Delta n(r, phi)$ im Material der Linse hergestellt.

Der oben bezeichnete Schritt 3, welcher die reale, körperliche Herstellung der erfindungsgemäßen Linse betrifft, beinhaltet das Herstellen der ersten optischen Fläche und der zweiten optischen Fläche sowie der spiralförmigen Brechkraftverteilung an und/oder auf und /oder innerhalb der Linse.

Die Herstellungsverfahren für optische Linsen, die im Zusammenhang mit der Erfindung auch mindestens eine nichtsphärische Fläche aufweisen kann, sind bekannt. Insbesondere sind dies:

    aa) Herstellung eines Höhenprofils der optischen Flächen durch Heißprägen oder Spritzgießen
    ab) Herstellung eines Höhenprofils der optischen Flächen durch Diamantdrehen
    ba) Herstellung einer diffraktiven Struktur durch lithographische Ätzverfahren auf der zweiten optischen Fläche
    bb) Herstellung einer diffraktiven Struktur durch Diamantdrehen auf der zweiten optischen Fläche
    ca) Herstellung eines Brechungsindex-Gradienten durch Schleuderguß aus dem flüssigen Zustand
    cb) Herstellung eines Brechungsindex-Gradienten durch Ionenimplantation.

Die Varianten a) und/oder b) können natürlich auch auf beiden optischen Flächen einer Linse in einer die Wirkung aufteilenden Weise angewendet werden. Diffraktive Optische Elemente können ergänzend oder zusammen mit der Erzeugung der Brechkraftverteilung zur Farbkorrektur eingesetzt werden. Zum Umfang der Erfindung gehören auch andere Verfahren und Maßnahmen, mit denen die erfindungsgemäße spiralförmige Brechkraftverteilung in einer Linse erzielt werden kann, beispielsweise durch Einbringen von Nanoteilchen.

Durch das beschriebene Vorgehen erreicht man zum Beispiel bei einer Intraokularlinse eine stetige Variation der zusätzlichen Brechkraft zur Brechkraft des Basissystems zwischen 0 und ca. 3.5 dpt bei einer in vielen Anwendungsfällen hinreichend guten Bildgüte.

Die radienabhängige und Azimut-winkelabhängige Brechkraft $F_G(r,phi)$ ergibt sich aus der Summe einer Grundbrechkraft des Basissystems $F_L$ und der radien- und winkelabhängigen Zusatzbrechkraft $F_S(r,phi)$:

$$F_G(r, phi) = F_L + F_S(r, phi) = \frac{1}{f_L} + \frac{1}{f_S(r, phi)}$$

Da standardisierte Optikverfahren zur Herstellung der Linse mit dem erweiterten Fokusbereich eingesetzt werden, ist diese Linse kostengünstig herstellbar.

Für den Fall a) "Aufaddieren" eines spiralförmigen Höhenprofils auf eine der optischen Flächen der Linse und damit die Realisierung einer spiralförmigen Brechkraftverteilung des Gesamtsystems gelten die nachfolgenden Betrachtungen:

Die Gesamtbrechkraft $F_G$ setzt sich aus einer Addition der Grundbrechkraft des Basissystems $F_L$ und der Zusatzbrechkraft $F_S$ zusammen.

$F_G(r,phi) = F_L + F_S(r,phi)$, wobei der spiralförmigen Brechkraftanteil $F_S(r,phi) = F_{Smax}(r,phi) * w(phi)$ ist.

Da in diesem Fall die Verteilung der Zusatzbrechkraft durch eine radiale Höhenverteilung erreicht wird, gilt

$$F_G(r, phi) = F_L + F_S(r, phi) => z_G(r, phi) = z_L(r) + z(r, phi).$$

Das Höhenprofil, welches die spiralförmige Zusatzbrechkraft liefert, ist allgemein durch $z(r,phi) = z_{max}(r,phi) * w(phi)$ beschrieben.

Die Grundbrechkraft des Basissystems ergibt sich für sphärische Linsen aus der Formel

$$F_L = \left[ \frac{n2 - n1}{n1} * \left( \frac{1}{R1} - \frac{1}{R2} \right) + \frac{(n2 - n1)^2 * d}{n1 * n2 * R1 * R2} \right].$$

Dabei ist beispielsweise $R_1$ der Radius der ersten optischen Fläche, welcher real hergestellt wird und $R_2$ ist der Radius der berechneten Basisfläche (die additive Höhe z, welche die Zusatzbrechkraft liefert, kann auch auf den Radius $R_1$ aufaddiert werden oder auf beide Radien $R_1$ und $R_2$ aufgeteilt werden, die Formeln müssen dann entsprechend verändert werden).

Das Höhenprofil $z_L$ für die errechnete Basisfläche mit dem Radius $R_2$ der sphärischen Linse ergibt sich zu

$$z_L(x, y) = R_2 - \sqrt{R_2^2 - x^2 - y^2}$$ , und mit $r = \sqrt{x^2 + y^2}$ ergeben sich die Daten der Basisfläche in Polarkoordinaten zu $z_L(r) = R - \sqrt{R^2 - r^2}$. Für den Fall einer sphärischen Basisfläche gilt daher

$$z_G(r, phi) = \left(R - \sqrt{R^2 - r^2}\right) + z_{max}(r, phi) * w(phi)$$

Sofern nichtsphärische Basisflächen für die Linse zu Grunde gelegt werden, werden die bekannten Polynome für die Beschreibung nichtsphärischer Flächen zur Bestimmung der optischen Flächen und/oder der Basisfläche verwendet. Die Zusatzbrechkraft wird hier im Fall a) durch einen additiven Term z(r, phi) als Materialhöhe erzeugt, welcher beispielsweise auf die optische Basisfläche mit dem Radius $R_2$ aufaddiert oder auch subtrahiert wird. Analoge Betrachtungen gelten auch für asphärische und Freiformflächen, die sich nicht mit einer einfachen Angabe eines Radius beschreiben lassen.

Das Radialpolynom für den maximalen Höhenanteil als Funktion des Radius r der Brechkraft $z_{max}(r)$, welches die maximale zu erzielende Dioptrienzahl verkörpert, ist:

$$z_{max}(r) = \sum_{j=2}^{N} c_j * r^j$$

$$w(phi) = \frac{phi}{2\pi}$$

mit r als radiale Höhe und $c_i$ : Koeffizientensatz des Radialpolynoms. $w(phi) = \frac{phi}{2\pi}$ ist der winkelabhängige, lineare normierte Anteil, mit phi als azimutalem Winkel auf der Basisfläche des Basissystems (Trägerlinse).

Der additive Term z(r,phi), welcher auf die Basisfläche der Linse addiert wird, ergibt sich aus

$$z(r, phi) = z_{max}(r) * w(phi) = \sum_{j=2}^{N} c_j * r^j * \frac{phi}{2\pi}$$

Im einfachsten Falle reicht es daher bereits aus, die zusätzliche radiale Brechkraftverteilung als Produkt des normierten Azimutwinkels mit der maximal zu erreichenden Dioptrienzahl zu realisieren.

Für das Radialpolynom $z_{max}(r)$ kann in analoger Weise auch der Ansatz $$z_{max}(r) = \sum_{j=1}^{N} c_j * r^{2*j}$$ verwendet werden.

Für den einfachsten Fall des Radialpolynoms $z_{max}(r) = c_1 * r^2$ mit $c_1$ als Koeffizienten vor dem quadratischen Term lautet die Gleichung für den additiven Term also

$$z(r, phi) = z_{max}(r) * w(phi) = c_1 * r^2 * \frac{phi}{2\pi}$$

Das obige beschriebene Vorgehen stellt einen linearen "Schraubenanstieg" dar. In dieser Form ist die Abbildungsqualität über den gesamten Dioptrienbereich in etwa gleichbleibend gut.

Oftmals ist es aber gewünscht, bestimmte Dioptrienbereiche wie zum Beispiel die Null-Dioptrien-Position zu bevorzugen. Hierzu ist es notwendig, die lineare Abhängigkeit der z-Höhe vom Winkel zu verlassen.

Der winkelabhängige Anteil kann allgemein durch die Formel

$$w(phi) = \sum_{i=1}^{M} I_i * \exp\left[-a_i * (phi - w_i)^2\right]$$

beschrieben werden, wobei $w_i$ die Peaklagen (zwischen 0 und $2\pi$), $I_i$ die Peakintensitäten und $a_i > 0$ die Dämpfungskoeffizienten für die jeweiligen Peaklagen sind.

Beispielsweise lässt sich für M=1; $I_1$=1 und $w_i$=$2\pi$ durch die Funktion

$$z(r, phi) = z_{\max}(r) * w(phi) = \sum_{j=2}^{N} c_j * r^j * \exp\left[-a_1 * (phi - 2\pi)^2\right]$$

mit $a_1 = 0.25$ eine Bevorzugung des Null-Dioptrienbereiches realisieren. Der geringe Anstieg zwischen phi $=0$ und phi $=2\pi$ verursacht eine geringe Addition von Brechkraft in diesem Winkelbereich und somit einen größeren Flächenanteil für die Null-Dioptrienentfernung.

Im Zusammenhang mit der Optimierung der Linse mit dem erweiterten Fokusbereich lassen sich weitere Vorteile erzielen, indem weitere Freiheitsgrade bei der Dimensionierung zur Verfügung stehen. Beispielsweise erfolgt dies, wenn die radiale Funktion $z_{\max}(r)$ ebenfalls einen azimutabhängigen Koeffizientensatz erhält und so das Radialpolynom $z_{\max}(r,$

$$z_{\max}(r, phi) = \sum_{j=2}^{N} c_j(phi) * r^j \qquad z_{\max}(r, phi) = \sum_{j=1}^{N} c_j(phi) * r^{2*j}$$

*phi*) bestimmt wird als $\qquad\qquad$ oder

Daraus ergibt sich der additive Term $z(r, phi)$ beispielsweise allgemein zu

$$z(r, phi) = z_{\max}(r, phi) * w(phi) = \sum_{j=2}^{N} c_j(phi) * r^j * \sum_{i=1}^{M} I_i * \exp\left[-a_i * (phi - w_i)^2\right]$$

Daher lassen sich für den Winkelterm $w(phi)$ noch weitere Varianten aus der allgemeinen Formel

$$w(phi) = \sum_{i=1}^{M} I_i * \exp\left[-a_i * (phi - w_i)^2\right]$$

heraus benennen, mit denen man die "Wirkungsdauer" der einzelnen Azimutbereiche steuern kann.

Die vorstehenden Ausführungen basierten auf einem additiven Term, welcher refraktiv ist und auf eine der optischen Flächen des Basissystems addiert wird. Der Additionsterm kann natürlich auch in diffraktiver Form vorliegen, d.h. auf die sphärische Trägerfläche des Basissystems wird ein Diffraktives Optisches Element (DOE) aufgebracht, welches eine spiralförmige Phasenfunktion besitzt (Fall b)). Die Gestaltung dieser Phasenfunktion erfolgt dabei in vollkommener Analogie zum refraktiven Ansatz. Es eignen sich insbesondere Blaze-Gitter, Sinusgitter und Binär-Gitter. Die Gitterfrequenz ändert sich radial und winkelabhängig spiralförmig stetig von einem Wert Null bis zu einem Maximal-

$$Phase_{\max}(r) = \sum_{j=2}^{N} k_j * r^j$$

wert, welcher der maximalen Brechkraft entspricht. $\qquad$ oder

$$Phase_{\max}(r) = \sum_{j=1}^{N} k_j * r^{2*j}$$

mit $k_1$ als Koeffizient des quadratischen Terms ergibt sich die maximale Brech-

$$F_{S\,\max\,diffraktiv} = 2k_1 \frac{\lambda}{wl}$$

kraft, $\qquad$ und der winkelabhängige Term $F_{Sdiffraktiv}(phi)$ ergibt sich zu

$$F_{S\,diffraktiv} = 2k_1 \frac{\lambda}{wl} * w(phi)$$

mit wl als Konstruktionswellenlänge des Diffraktiven Optischen Elements und $\lambda$ als Anwendungswellenlänge.

Die Gesamtbrechkraft der Linse ergibt sich aus einer vergleichsweise starken refraktiven Grundbrechkraft des monofokalen Basissystems und aus einem relativ kleinen Brechkraftanteil der diffraktiv erzeugten Zusatzbrechkraft:

$$F_{G\,diffraktiv} = F_L + F_{S\,diffraktiv} = F_L + 2k_1 \frac{\lambda}{wl} * w(phi).$$

So entsteht durch den diffraktiven Anteil ein relativ nur kleiner Farbfehler und die Linse mit dem erweiterten Fokusbereich ist auch für weißes Licht geeignet.

Der Additionsterm kann jedoch auch durch die Herstellung eines spiralförmigen Brechzahlgradienten realisiert werden (Fall c)). In DE 10 2009 033 984 A1 ist beschrieben, wie in einem optischen Material inhomogene optische Eigenschaften erzeugbar sind. In Abwandlung des dort beschriebenen Verfahrens lässt sich auch ein spiralförmiger Brechungsindex-Verlauf realisieren. Die Eigenschaften und die Gestaltung des Brechzahlgradienten erfolgt dabei in vollkommener Analogie zum refraktiven Ansatz.

Die Gesamtbrechkraft $F_G$ ergibt sich aus der Grundbrechkraft $F_L$ des monofokalen Basissystems plus der Zusatzbrechkraft $F_S$, welche durch die spiralförmige Brechkrafterhöhung bereitgestellt wird.

$$F_G = F_L + F_S(r, phi) = \frac{1}{f_L} + \frac{1}{f_S(r, phi)},$$

wobei $f_L$ die Brennweite des Basissystems ist und $f_S$ die Brennweite der spiralförmigen Zusatzbrechkraft, welche durch den Brechungsindexgradienten hervorgerufen wird. Die Zusatzbrechkraft $F_S$ (r, phi) ist proportional der Brechungsindexdifferenz $\Delta n$(r, phi) gemäß der Formel

$$\Delta n(r, phi) = \Delta n_{max}(r) * w(phi)$$

oder

$$\Delta n(r, phi) = \Delta n_{max}(r, phi) * w(phi).$$

Die Brechungsindexdifferenz $\Delta n$(r, phi) läuft stetig von 0 (bei r=0 und phi=0) bis zur maximalen Brechungsindexerhöhung $\Delta n_{max}$ (bei r=D/2 und phi=$2\pi$), wobei die Funktion w(phi) den oben beschriebenen linearen oder allgemeinen Verlauf vorgeben kann.

Dabei kann $\Delta n_{max}$ gegenüber der Basisbrechzahl $n_2$ der Linse sowohl positiv als auch negativ sein.

Die zuvor beschriebenen Verfahren werden eingesetzt um die spiralförmige Brechkraftverteilung $F_S$(r,phi) an und/oder in eine Augenlinse, eine phake Intraokularlinse oder eine aphake Intraokularlinse aufzuprägen und/oder einzuprägen.

Die Erfindung betrifft weiterhin ein Objektiv mit einem erweiterten Fokusbereich, welches im Strahlengang eine erfindungsgemäße Linse mit einem erweiterten Fokusbereich als abbildendes Element aufweist.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend an Hand von Figuren beschrieben. Es zeigen:

Figur 1: eine Linse mit erweitertem Fokusbereich in der Draufsicht in einem Graustufenbild mit dem spiralförmigen Brechkraftverlauf;

Figur 2: eine Linse mit erweitertem Fokusbereich in der Seitenansicht mit Darstellung des spiralförmigen refraktiven Anteils;

Figur 3: ein optisches System einer Kamera mit einer Linse mit dem erweiterten Fokusbereich;

Figur 4: eine schematische Darstellung einer Intraokularlinse im Auge;

Figur 5: Azimutaler Verlauf des spiralförmigen aufmodulierten Anteils des Brechkraftverlaufes mit vorrangig linearem Anstieg;

Figur 6: Azimutaler Verlauf des spiralförmigen aufmodulierten Anteils des Brechkraftverlaufes mit Bevorzugung des Null-Dioptrienbereiches;

Figur 7: Erweiterte Fokuslinsen-Oberfläche mit aufmoduliertem spiralförmigen Höhenprofil, stark überhöhte Darstellung;

Figur 8: Linse mit erweitertem Fokusbereich in der Draufsicht mit Darstellung des spiralförmigen diffraktiven Anteils;

Figur 9: Linse mit erweitertem Fokusbereich in einer perspektivischen Draufsicht mit Darstellung des spiralförmigen diffraktiven Anteils;

Figur 10: Azimutaler Verlauf der Zusatzbrechkraft mit ausgedehntem Bereich für die starke Dioptrienlage; und

Figur 11: Azimutaler Verlauf der Zusatzbrechkraft mit Bevorzugung des Null-Dioptrienbereiches.

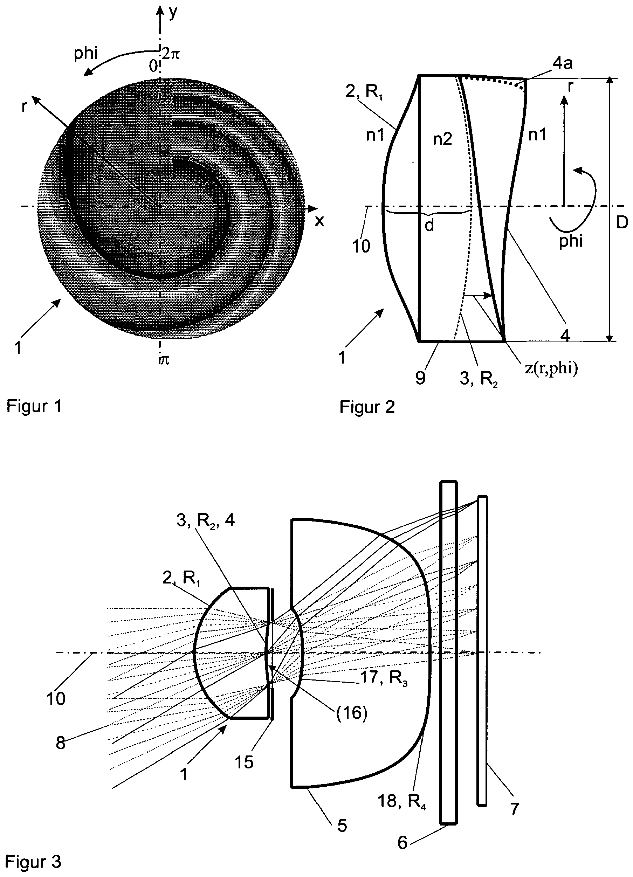

Figur 1 zeigt eine Linse mit einem erweiterten Fokusbereich mit dem spiralförmigen Brechkraftverlauf in der Draufsicht als Stufenbild der Brechkraftänderung. Die Darstellung gilt prinzipiell für die Fälle:

> a) "Aufaddieren" eines spiralförmigen Höhenprofils auf eine der optischen Flächen der Linse gemäß Schritt 1, welche die Basisfläche ist.
> b) "Aufaddieren" einer spiralförmigen diffraktiven Struktur auf eine der optischen Flächen der Linse gemäß Schritt 1, welche die Basisfläche ist.
> c) "Aufaddieren" eines spiralförmigen Brechungsindexverlaufes im Material der Linse.

Im Beispiel hat die Linse herkömmliche sphärische optische Flächen und eine Linsendicke, die ein Basissystem bilden, welches mit einer "Basis-Brechzahl" von 1,5995 für 0 dpt Korrektur ausgelegt ist. Die zusätzliche spiralförmige Brechkraftverteilung wird durch einen spiralförmigen Brechungsindexgradienten realisiert und beginnt bei phi=0 mit der Brechzahl von 1,5995. Die Brechzahl steigt radial- und winkelabhängig stetig spiralförmig und hat beispielsweise bei phi=$\pi$ und r= D/2 eine Brechzahl von 1,61366. Das entspricht einer Brechkraft von 1,0 dpt. Die Brechzahl steigt weiter stetig an und hat bei phi=2 $\pi$ und r= D/2 eine Brechzahl von 1,64615, was einer Brechkraft von 3,5 dpt entspricht. Zwischen phi=0 und phi=2 $\pi$ beträgt die Brechzahldifferenz 0,04665. Das entspricht einem nutzbaren kontinuierlichen Fokusbereich zwischen 0 dpt und 3,5 dpt.

Figur 2 zeigt eine Linse mit einem erweiterten Fokusbereich in der Seitenansicht mit Darstellung des spiralförmigen refraktiven Anteils. Die Linse 1 ist zunächst durch ihr Basissystem mit dem Radius $R_1$ der ersten optischen Fläche 2 und dem Radius $R_2$ für die errechnete Basisfläche 3, sowie der Linsendicke d und der Brechungsindex $n_2$ bestimmt. Diese Parameter sind für eine vorgesehene Grundvergrößerung bestimmt. Auf die errechnete Form der Basisfläche 3 mit dem Radius $R_2$ wird eine zusätzliche Materialdicke z "aufaddiert", wobei bei phi=0 die zusätzliche Materialdicke z=0 mm ist, dann stetig ansteigt und bei phi=2 $\pi$ seinen maximalen Wert im Millimeterbereich hat. In der Praxis wird der Maximalwert etwas vor dem Azimutwinkel phi=2 $\pi$ liegen, um einen stetigen, wenn auch sehr steilen Übergang zurück zum Wert Null bei phi=0 zu realisieren, wie das durch die mit 4a bezeichnete gestrichelte Kurve angedeutet ist.

Als Beispiel werden Parameter für eine Linse angegeben:

> $R_1$= -15,1411 mm herzustellender Radius
> $R_2$= 22,3164 mm berechneter Radius
> d= 0,8 mm
> $n_1$= 1 (Brechzahl außerhalb der Linse)
> $n_2$= 1,56 (Brechzahl des Linsenmediums)

Somit ergibt sich aus der Formel

$$f = \cfrac{1}{\left[\cfrac{n2-n1}{n1} * \left(\cfrac{1}{R1} - \cfrac{1}{R2}\right) + \cfrac{(n2-n1)^2 * d}{n1 * n2 * R1 * R2}\right]}$$

die Brennweite der "Basislinse" zu 16,233 mm.
Auf die errechnete Basisfläche mit dem Radius $R_2$= 22,3164 mm wird ein linearer Schraubenanstieg gemäß der Formel

$$z(r, phi) = z_{\max}(r) * w(phi) = c_1 * r^2 * \frac{phi}{2\pi}$$

als stetiges, linear verlaufendes spiralförmiges Höhenprofil aufaddiert. Mit $c_1$ =-0,013 erhält man einen spiralförmigen Zusatz, welcher die Brennweite in Luft bis auf 20,57 mm erhöht, was 3,5 dpt entspricht.

Figur 3 zeigt ein optisches System einer Kamera mit einer erfindungsgemäßen Linse 1, welche den erweiterten Fokusbereich aufweist. Das optische System besteht aus der erweiterten Fokuslinse 1, und in Lichtausbreitungsrichtung folgt eine asphärische Linse 5 mit den optischen Flächen 17 und 18, dann folgen ein Filter 6 und ein Sensor 7. Die erweiterte Fokuslinse 1 hat objektseitig eine erste optische Fläche 2. Bildseitig ist die spiralförmig ausgebildete zweite optische Fläche 4 angeordnet.

Als Beispiel ist ein Objektiv eines Mobiltelefons mit einer Brennweite von 5,61 mm gezeigt. Gemäß der Formel

$$z(r, phi) = c_1 * r^2 * \frac{phi}{2\pi}$$

für den spiralförmigen, linearen Schraubenanstieg ergibt sich mit den Parametern $c_1$ =-0,01; phi = 0 bis $2\pi$ (Azimutwinkel), r = radiale Höhe zwischen 0 und D/2, die Form der spiralförmigen optische Fläche 4 über der berechneten Basisfläche 3 in Analogie wie zu Figur 2 beschrieben. Die Basisfläche 3 ist im Beispiel konkav und sphärisch, mit dem Radius $R_2$ = 5,21369 mm.

Die optischen Flächen 17 und 18 der Linse 5 sowie die erste optische Fläche 2 der erweiterte Fokuslinse 1 sind rotationsasphärisch. Die Parameter des Objektivs sind: Brennweite f= 5,61 mm; Baulänge 6,8 mm; Öffnung 1:2.8.

Linse 1:

Dicke 1,738 mm; Material = Zeonex
Erste optische Fläche 2: $R_1$= 1,7668 mm
Asphärenkoeffizienten:

K = -0,162288
A = 0,472171E-04
B = 0,225901E-02
C = -0,179019E-03
D = -0,290228E-03
E = 0,131193E-03

Zweite optische Fläche 4

Der berechnete Radius der Basisfläche 3 für die Grundbrechkraft der Linse $F_L$ ist $R_2$ = 5,21369 mm (konkav, sphärisch). Die Grundbrennweite der Linse ist 330 mm, was einer Zusatzbrechkraft von maximal 3,0 dpt entspricht.

Die Oberflächenform der Basisfläche kann mit der Formel $z_L(r) = R_2 - signum(R_2)\sqrt{R_2^{\,2} - r^2}$ beschrieben werden. Die additive Höhe ergibt sich aus $z(r, phi) = z_{max}(r) * w(phi) = c_1 * r^2 * \frac{phi}{2\pi}$ mit dem Koeffizient des Polynoms $c_1$ = -0,01.

Für jeden Flächenpunkt der Basisfläche ist die additive Höhe hinzuzurechnen, so dass das Gesamthöhenprofil der zweiten optischen Fläche durch folgende Formel bestimmt ist:

$$z_G(r, phi) = \left( R_2 - signum(R_2)\sqrt{R_2^{\,2} - r^2} \right) + c_1 * r^2 * \frac{phi}{2\pi}$$

Linse 5:

Dicke 2,703 mm; Material = Polycarbonat
Fläche 17: R = -3,85282 mm
Asphärenkoeffizienten:

K = 16,027906

A = -0,687655E-01

B = 0,676838E-01

C = -0,101439E+00

D = 0,900331E-02

E = 0,345714E-01

F = -0,101087E-01

G = 0,950453E-16

H = 0,443668E-17

J = 0,105965E-19

Fläche 18: $R_4$ = 413.75417 mm
Asphärenkoeffizienten:

K = -0.238656e57

A = -.200963E-01

B = 0.297531E-02

C = -.110276E-02

D = 0.209745E-03

E = -.935430E-05

F = -.430237E-05

G = 0.434653E-06

H = 0.475646E-07

J = -.612564E-08

| | |
|---|---|
| Abstand Linse 1 bis zur Linse 5 : | 0,571 mm |
| Abstand Linse 5 bis zum Filter 6: | 0,4 mm |
| Abstand Filter bis zur Bildebene des Detektors 7: | 0,4 mm |
| Dicke des Filters 6 | 0,4 mm |

Das optische System hat eine Baulänge von 6,8 mm. Die Öffnung beträgt 1:2,8. Das Objektiv liefert für einen Objektabstand von 330 mm bis unendlich eine ausreichend gute Bildqualität, welche ohne Nachfokussieren erreicht wird. Es ist vorteilhaft, dass sich die spiralförmige optische zweite Fläche auf der Rückseite der Frontlinse befindet, wobei die übrige Fläche der Rückseite, welche nicht von der spiralförmigen optischen Fläche ausgefüllt ist, eine Blende 15 bildet.

Ein weiteres Ausführungsbeispiel beschreibt ein Objektiv für eine Kamera mit einer Brennweite von f= 6,1 mm, bei einer Baulänge von 6,8 mm und einer Öffnung von 1:2,8. Die Darstellung entspricht der in Figur 3 gezeigten. Die Linse 1 mit dem erweiterten Fokusbereich hat objektseitig eine erste optische Fläche 2. Ihre zweite optische Fläche 4 entspricht in ihrer Oberflächenform der errechneten Basisfläche 3 und trägt das Diffraktive Optische Element 16, welches den spiralförmigen Brechkraftverlauf zusätzlich zur Brechkraft des Basissystems liefert.

Alle optischen Flächen 3, 4, 17 und 18 der Linsen 1 und 5 besitzen eine rotationsasphärische Grundform.
Linse1: Linsendicke = 1,59mm, Material = Zeonex
optische Fläche 2: $R_1$ = 1,77985
Asphärenkoeffizienten:

K = 0,113528
A = -0,369422E-02
B = 0,497838E-05
C = -0,526491E-03

optische Fläche 4 (entspricht der errechneten Fläche 3): $R_2$ = 4,43773

Asphärenkoeffizienten:

K = 20,010847
A = -0,165668E-01
B = 0,598703E-01
C = -0,239849E+00
D = 0,363395E+00
E = -0,231421E+00

Das Diffraktive Optische Element 16 hat den Koeffizienten des spiralförmigen Polynoms $k_1$ = -2,1350E-03
Der zusätzliche spiralförmige Brechkraftanteil berechnet sich zu

$$F_{S\ diffraktiv} = 2k_1 * \frac{phi}{2\pi} * \frac{\lambda}{wl}$$

Und die Gesamtbrechkraft ergibt sich zu

$$F_{G\ diffraktiv} = F_L + F_{S\ diffraktiv} = F_L + 2k_1 * \frac{phi}{2\pi} * \frac{\lambda}{wl}$$

Die Linse 5 hat eine Dicke von 2,98 mm, Material = Polycarbonat Fläche 17:_$R_3$ = -4,60229 mm
Asphärenkoeffizienten:

K = 12.980316
A = -.289939E-01
B = -.193341E-01
C = 0.430879E-01
D = -.575934E-01
E = 0.345714E-01
F = -.101087E-01

Fläche 18: $R_4$ = -51,75016 mm
Asphärenkoeffizienten:

K = -0,238656e57
A = -0,128992E-01
B = 0,257544E-02
C = -0,116486E-02
D = 0,176791E-03
E = -0,381907E-06
F = -0,294503E-05
G = 0,250155E-06
H = 0,303670E-08
J = -0,768736E-09

Der Abstand zwischen der Linse 1 und der Linse 5 beträgt 1,05 mm, der Abstand zwischen der Linse 5 und dem Filter 6 beträgt 0,4 mm und der Abstand vom Filter 6 bis zur Bildebene des Detektors 7 beträgt 0,4 mm, wobei die Filterdicke ebenfalls 0,4 mm beträgt.

Das Objektiv liefert einen gleichzeitigen Fokusbereich von 330 mm bis unendlich.

Dabei unterstützt insbesondere die zweckmäßige Wahl des Koeffizienten c vor dem quadratischen Term die Achromatisierung des Objektivs.

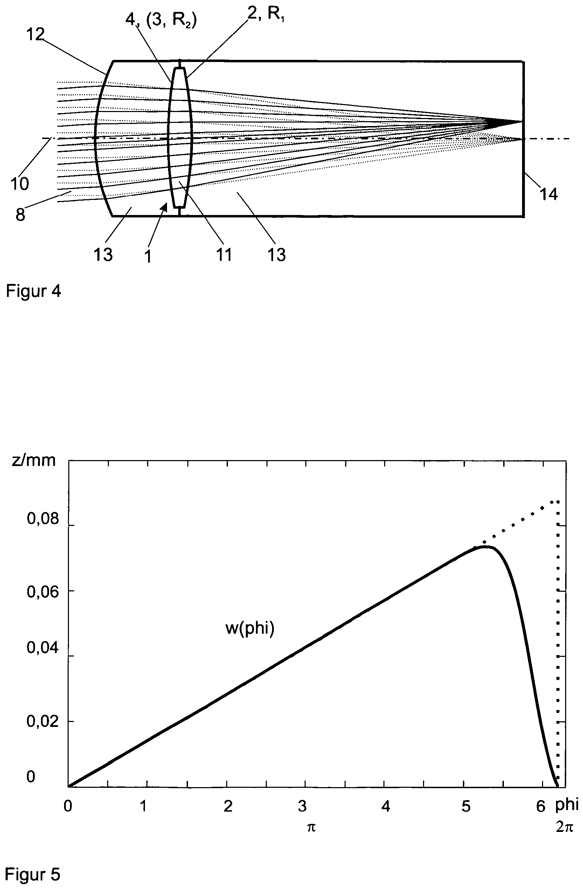

Figur 4 zeigt eine schematische Darstellung einer Intraokularlinse 11, welche als erweiterte Fokuslinse 1 ins Auge implantiert ist. Im Beispiel ersetzt diese die natürliche Augenlinse und befindet sich im Lichtweg zwischen der Cornea 12 und der Retina 14 im Kammerwasser 13.

Die Intraokularlinse 11 hat eine sphärische erste optische Fläche 2 sowie die spiralförmige zweite optische Fläche 4. Beispielsweise hat die Intraokularlinse 11 mit dem erweiterten Fokusbereich folgende Parameter für das Basissystem: $R_1$= -15,1411 mm (hergestellte erste optische Fläche 2)

$R_2$= 22,3164 mm (berechnete Basisfläche 3)

Linsendicke d= 0,8 mm

Brechzahl außerhalb der Linse $n_1$= 1,33

Brechzahl des Linsenmediums $n_2$= 1,56

Mit der Formel

$$f = \cfrac{1}{\left[\dfrac{n2-n1}{n1} * \left(\dfrac{1}{R1} - \dfrac{1}{R2}\right) + \dfrac{(n2-n1)^2 * d}{n1 * n2 * R1 * R2}\right]}$$

ergibt sich die Basis-Brennweite f= 53,97 mm für das Basissystem der Intraokularlinse 11 im Kammerwasser 13.

Die Zusatzbrechkraft ergibt sich aus der additiven Höhe auf der Basisfläche mit der Formel

$$z(r, phi) = c_1 * r^2 * \frac{phi}{2\pi} \quad \text{mit } c_1 = -0,013$$

Die "aufaddierte" Spiralfläche würde den Wert der Basisbrennweite von 16,233 mm auf 17.2 mm verlängern, was 3.5 dpt entspricht. Die erweiterte Fokuslinse liefert demnach eine Varianz des Dioptrienbereiches zwischen 0 dpt und 3,5 dpt.

Figur 5 zeigt den azimutalen Verlauf des spiralförmigen aufmodulierten Anteils des Brechkraftverlaufes auf die Basisfläche mit einem vorrangig linearen Anstieg entsprechend der Formel

$$z(r, phi) = z_{max}(r) * w(phi) = c_1 * r^2 * \frac{phi}{2\pi}$$

für den additiven Anteil an der Brechkraft.

Aus Gründen der besseren Herstellbarkeit und um scharfe Übergänge zu vermeiden ist der Kurvenverlauf in der Nähe von $2\pi$ geglättet.

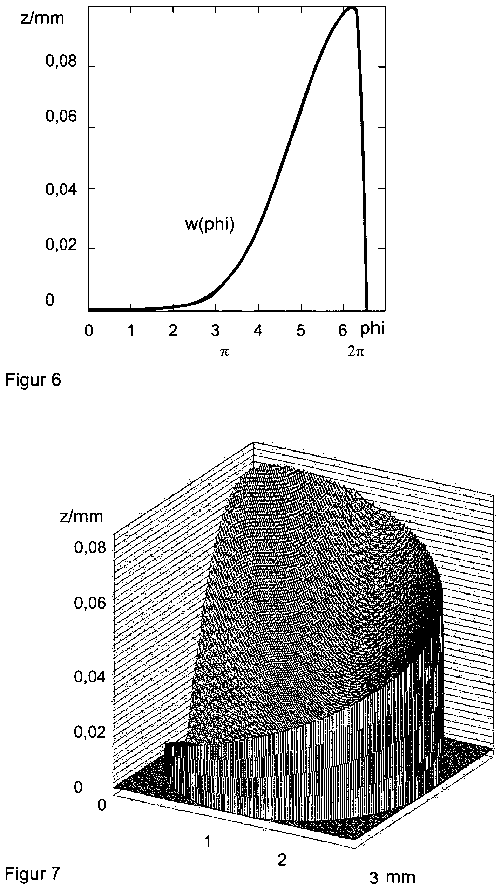

Figur 6 zeigt den azimutalen Verlauf des spiralförmigen aufmodulierten Anteils des Brechkraftverlaufes mit Bevorzugung des Null-Dioptrienbereiches In der Praxis ist es oftmals gewünscht, bestimmte Dioptrienbereiche wie zum Beispiel die Null-Dioptrien-Position zu bevorzugen. Hierzu ist es notwendig, die lineare Abhängigkeit der z-Höhe vom Winkel zu verlassen. Beispielsweise lässt sich durch die Funktion

$$z(r, phi) = z_{max}(r) * w(phi) = \sum_{j=1}^{N} c_j * r^{2*j} * \exp\left[-a * (phi - 2\pi)^2\right]$$

mit a=0.25 eine Bevorzugung des Null-Dioptrienbereiches verwirklichen.

Der winkelabhängige Anteil $w(phi) = \exp\lfloor - a * (phi - 2\pi)^2 \rfloor$

ist in Figur 6 dargestellt. Der geringe Anstieg zwischen

phi =0 und phi =$2\pi$ verursacht eine geringe Addition von Brechkraft in diesem Winkelbereich und somit einen größeren Flächenanteil für die

Nulldioptrienentfernung.

Figur 7 zeigt eine überhöhte Darstellung des spiralförmig verlaufenden additiven Höhenprofils gemäß der Figur 2. Die spiralförmige optische Fläche 4 entsteht dadurch, dass auf die sphärische Basisfläche 3 die azimutal-abhängige Polynomfunktion aufaddiert ist. Dargestellt ist die auf die sphärische Basisfläche 3 gemäß Figur 2 rein aufaddierte Höhe z über dem Durchmesser der Linsenoberfläche.

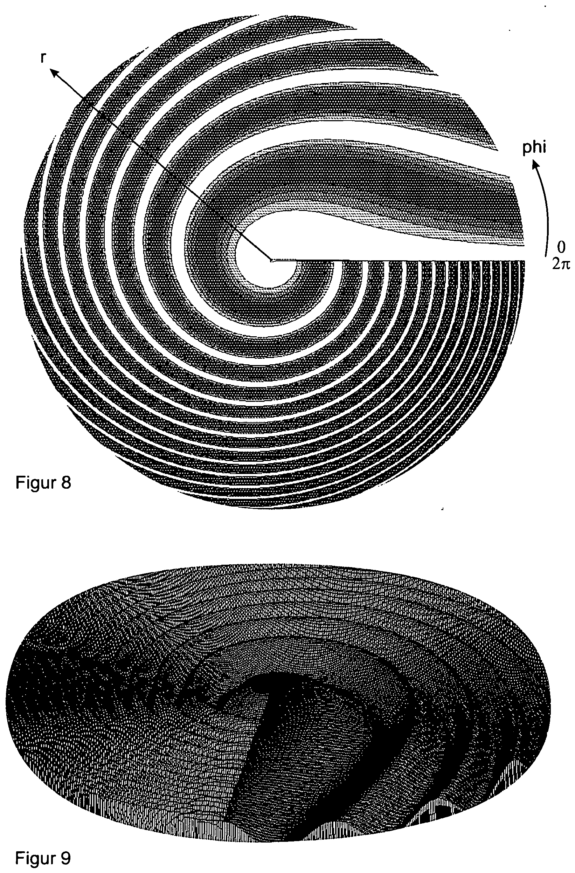

Figur 8 zeigt eine Linse mit einem erweiterten Fokusbereich, welche nach dem diffraktiven Ansatz gemäß Variante b) berechnet wurde in der Draufsicht, wobei nur der spiralförmige diffraktive Anteil sichtbar ist. In diesem Fall der Erzeugung der spiralförmigen Brechkraftverteilung der Linse mittels des diffraktiven Ansatzes lautet die Phasenfunktion:

$$Phase(r, phi) = Phase_{max}(r) * w(phi) = \left\langle \sum_{j=2}^{N} k_j * r^j \right\rangle * \left\langle \sum_{i=1}^{M} I_i * \exp\left[- a_i * (phi - w_i)^2 \right] \right\rangle$$

Mit

$$t = \sum_{j=2}^{N} k_j * r^j * \sum_{i=1}^{M} I_i * \exp\left[- a_i * (phi - w_i)^2 \right]$$

ergibt sich das Profil (r, phi) zu

$$\mathrm{Pr}ofil(r, phi) = \left( \frac{t}{wl} - floor\left( \frac{t}{wl} \right) \right) * h$$

$$\mathrm{Pr}ofil(r, phi) = \left( \frac{\sum_{j=2}^{N} k_j * r^j * \sum_{i=1}^{M} I_i * \exp\left[- a_i * (phi - w_i)^2 \right]}{wl} - floor\left( \frac{\sum_{j=2}^{N} k_j * r^j * \sum_{i=1}^{M} I_i * \exp\left[- a_i * (phi - w_i)^2 \right]}{wl} \right) \right) * h$$

mit $k_i$ Koeffizient des diffraktiven Polynoms; r Radius (radiale Höhe); $I_i$ Intensitäten; $a_i$ Dämpfungskoeffizienten; wl Konstruktionswellenlänge des DOE und h Profiltiefe.

Für den linearen Spezialfall gilt:

$$Phase(r, phi) = Phase_{max}(r) * w(phi) = k_1 * r^2 * \frac{phi}{2\pi}.$$

Mit

$$t = k_1 * r^2 * \frac{phi}{2\pi}$$

ergibt sich

$$\mathrm{Pr}\,ofil(r, phi) = \left(\frac{t}{wl}t - floor\left(\frac{t}{wl}\right)\right) * h =$$

$$\left(\frac{k_1 * r^2 * phi}{wl * 2\pi} - floor\left(\frac{k_1 * r^2 * phi}{wl * 2\pi}\right)\right) * h$$

Beispieldaten sind: Koeffizient des diffraktiven Polynoms $k_1 = 0.0025$, die Höhe r im Bereich zwischen 0 und 3 mm, der Azimutwinkel phi im Bereich von 0 bis $2\pi$, Konstruktionswellenlänge des DOE wl= 550nm, Profiltiefe h= 0.001mm.

Figur 9 zeigt die Linse mit dem erweiterten Fokusbereich in einer perspektivischen Draufsicht auf den Durchmesser der Linse mit Darstellung des spiralförmigen diffraktiven Anteils.

Mit der modifizierten Formel für die Winkelabhängigkeit

$$w(phi) = I_1 * \exp\left[-a_1 * (phi - w_1)^2\right] + I_2 * \exp\left[-a_2 * (phi - w_2)^2\right]$$

wobei

$w_1, w_2$ : Peak-Lagen (zwischen 0 und 2*pi),
$I_1, I_2$ : Peakintensitäten und
$a_1, a_2$: Dämpfungskoeffizienten für die beiden Peaks
sind, ergibt sich beispielsweise mit den Werten
$I_1 = 0,9$; $I_2 = 0,1$; $a_1 = 0,8$; $a_2 = 0,1$; $w_1 = 1,8$; $w_2 = 4,5$
ein Verlauf für die Azimutabhängigkeit, bei dem ein ausgedehnter Bereich für die stärkste Dioptrienlage (ca. 3dpt) reserviert wird.

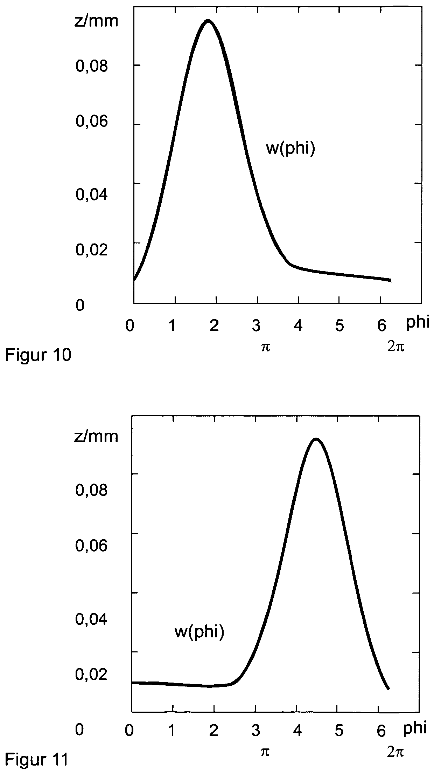

Mit den Werten $I_1 = 0,9$; $I_2 = 0,1$; $a_1 = 0,8$; $a_2 = 0,1$; $w_1 = 4,5$; $w_2 = 0,5$ wird mit der Formel $w(phi) = I_1 * \exp[-a_1 * (phi - w_1)^2] + I_2 * \exp[-a_2 * (phi - w_2)^2]$ der Null-Dioptrienbereich deutlich bevorzugt, was in Figur 11 dargestellt ist.

## Bezugszeichen

| | |
|---|---|
| 1 | Linse |
| 2 | gefertigte erste optische Fläche (sphärisch, asphärisch, radialsymmetrisch, Freiformfläche) |
| 3 | berechnete Basisfläche (sphärisch, asphärisch, radialsymmetrisch, Freiformfläche) |
| 4 | gefertigte zweite optische Fläche (sphärisch, asphärisch, radialsymmetrisch, Freiformfläche, spiralförmige Oberfläche) |
| 5 | asphärische Linse |
| 6 | Filter |
| 7 | Sensor |
| 8 | Lichtbündel |
| 9 | Linsenrand |
| 10 | optische Achse |
| 11 | Intraokularlinse |
| 12 | Cornea |
| 13 | Kammerwasser |
| 14 | Retina |
| 15 | Blende |
| 16 | spiralförmiges Diffraktives Optisches Element (DOE) |
| 17 | optische Fläche |
| 18 | optische Fläche |
| $F_G$ | Gesamtbrechkraft der Linse |

| | |
|---|---|
| $F_L$ | Brechkraft des Basissystems der Linse |
| $F_S(r, phi)$ | Brechkraft, die durch den spiralförmigen Anteil zur Brechkraft des Basissystems hinzu kommt |
| $F_{S\,max}$ | maximale Brechkraft |
| $f_L$ | Brennweite des Basissystems |
| $f_S(r, phi)$ | Brennweite der spiralförmigen Zusatzbrechkraft |
| N, M | Endwerte |
| i, j | Zählwerte |
| $c_j, c_1, c_2$ | Polynomkoeffizienten für den refraktiven Fall |
| $k_j, k_1, k_2$ | Polynomkoeffizienten für den diffraktiven Fall |
| $z_{max}(r)$ | maximale Höhe in Abhängigkeit vom Radius |
| $z_{max}(r, phi)$ | maximale Höhe, in Abhängigkeit vom Radius und Azimutwinkel |
| $z(r, phi)$ | additive Höhe auf der Basisfläche |
| $z_L(r)$ | Höhenprofil der berechneten Basisfläche |
| $z_G(r, phi)$ | Höhenprofil der gefertigten optischen Fläche |
| $w(phi)$ | winkelabhängiger Anteil des Brechkraftverlaufes |
| $w_i, w_1, w_2$ | Peaklagen der Winkelverteilungsfunktion |
| $a_i, a_1, a_2$ | Dämpfungskoeffizienten für die jeweiligen Peaklagen |
| $I_i, I_1, I_2$ | Intensitätswerte der Einzelpeaks |
| D | Linsendurchmesser |
| r | Radius (radiale Höhe) |
| phi | Azimutwinkel |
| $R_1$ | Radius der ersten optischen Fläche |
| $R_2$ | Radius der optischen Basisfläche |
| $n_1$ | Brechzahl des umgebenden Mediums |
| $n_2$ | Brechzahl der Linse |
| d | Linsendicke |
| h | Profiltiefe des diffraktiven Elements |
| $\lambda$ | Anwendungswellenlänge |
| wl | Konstruktionswellenlänge des diffraktiven Elements |
| t | Rechengröße |
| floor(t) | ganzzahliger Anteil |
| $Phase_{max}(r, phi)$ | Maximalwert der Gitterfrequenz, welcher der maximalen Brechkraft entspricht |
| Phase(r, phi) | Phasenfunktion |
| Profil(r, phi) | auf die Höhe h reduzierte Phasenfunktion |
| x, y | kartesische Koordinaten |

**Patentansprüche**

1. Linse mit einem erweiterten Fokusbereich, wobei die Linse (1) aus einem festen, transparenten Material besteht sowie zwei gefertigte optische Flächen (2, 4) hat, wobei die Linse (1) eine Brechkraftverteilung $F_G$ aufweist, **dadurch gekennzeichnet, dass**
sich die Brechkraftverteilung $F_G$ der Linse (1) bezogen auf eine zur optischen Achse (10) senkrecht stehende Ebene als Funktion der radialen Höhe r und des Azimutwinkels phi der Apertur zwischen einem Grundwert der Brechkraft $F_L$ ungleich Null und einem Maximalwert $F_{Smax}$ ungleich Null ändert und somit sich die Brechkraftverteilung zu $F_G(r,phi) = F_L + F_S(r,phi)$ ergibt, mit einem spiralförmigen Brechkraftanteil $F_S(r,phi) = F_{Smax}(r) * w(phi)$
wobei $F_{Smax}(r)$ nichtlinear radienabhängig ist und w(phi) ein Faktor für den azimutalen Brechkraftanteil mit dem

$$w(phi) = \sum_{i=1}^{M} I_i \exp\left[-a_i(phi - w_i)^2\right]$$

spiralförmigen Verlauf ist, der mit der Formel zur Addition von Brechkraft im Azimutwinkel phi, der Werte von Null bis $2\pi$ annimmt, beschrieben ist, und $w_i$ die Peaklagen der Winkelverteilungsfunktion; $I_i$ Intensitätswerte der Einzelpeaks mit $I_i > 0$; $a_i > 0$ Dämpfungskoeffizienten für die jeweiligen Peaklagen sowie i ein Zählwert und $M \geq i$ ein Endwert sind.

2. Linse mit einem erweiterten Fokusbereich, wobei die Linse (1) aus einem festen, transparenten Material besteht sowie zwei gefertigte optische Flächen (2, 4) hat, wobei die Linse (1) eine Brechkraftverteilung $F_G$ aufweist, **dadurch**

**gekennzeichnet, dass**

sich die Brechkraftverteilung $F_G$ der Linse (1) bezogen auf eine zur optischen Achse (10) senkrecht stehende Ebene als Funktion der radialen Höhe r und des Azimutwinkels phi der Apertur zwischen einem Grundwert der Brechkraft $F_L$ ungleich Null und einem Maximalwert $F_{Smax}$ ungleich Null ändert und somit sich die Brechkraftverteilung zu $F_G(r,phi) = F_L + F_S(r, phi)$ ergibt, mit einem spiralförmigen Brechkraftanteil

$$F_S(r, phi) = F_{S\max}(r) * w(phi)$$

wobei $F_{Smax}(r)$ nichtlinear radienabhängig ist und w(phi) ein Faktor für den Brechkraftanteil mit dem spiralförmigen

$$w(phi) = \frac{phi}{2\pi}$$

Verlauf ist, der mit der Formel $\phantom{x}$ als linearer Verlauf beschrieben ist für Werte vom Azimutwinkel phi von Null bis $2\pi$.

3. Linse nach Anspruch 1 oder nach Anspruch 2, **dadurch gekennzeichnet, dass** die maximale Brechkraft $F_{Smax}(r)$

$$F_{S\max}(r) = \sum_{j=2}^{N} c_j * r^j$$

nichtlinear radienabhängig ist und mit den Polynomformeln $\phantom{xxxxxx}$ oder

$$F_{S\max}(r) = \sum_{j=1}^{N} c_j * r^{2*j}$$

mit den Polynomkoeffizienten $c_j$ für eine refraktive Brechkraft und

$$F_{S\max}(r) = \sum_{j=2}^{N} k_j * r^j \qquad F_{S\max}(r) = \sum_{j=1}^{N} k_j * r^{2*j}$$

oder $\phantom{xxxxxx}$ mit dem Polynomkoeffizienten $k_j$ für eine diffraktive Brechkraft beschrieben ist, wobei j ein Zählwert und N$\geq$j ein Endwert sind.

4. Linse nach Anspruch 1 oder nach Anspruch 2, **dadurch gekennzeichnet, dass** die maximale Brechkraft $F_{Smax}(r,phi)$ nichtlinear radienabhängig und vom Azimutwinkel phi der Apertur abhängig ist und durch die Polynomformeln

$$F_{S\max}(r, phi) = \sum_{j=2}^{N} c_j(phi) * r^j \qquad F_{S\max}(r, phi) = \sum_{j=1}^{N} c_j(phi) * r^{2*j}$$

oder $\phantom{xxxxxx}$ mit den Polynomkoeffizienten

$$F_{S\max}(r, phi) = \sum_{j=2}^{N} k_j(phi) * r^j$$

$c_j$ für eine refraktive Brechkraft und $\phantom{xxxxxx}$ oder

$$F_{S\max}(r, phi) = \sum_{j=1}^{N} k_j(phi) * r^{2*j}$$

mit dem Polynomkoeffizienten $k_j$ für eine diffraktive Brechkraft beschrieben ist, wobei j ein Zählwert und N$\geq$j ein Endwert sind.

5. Linse nach Anspruch 3, **dadurch gekennzeichnet, dass** die Brechkraftverteilung $F_G(r,phi)$ der Linse (1) sich aus einem Höhenprofil $z_G(r, phi)$ einer zu fertigenden zweiten optischen Fläche (4) ergibt, welches sich aus der Addition des Höhenprofils $z_L(r)$ einer berechneten Basisfläche (3) und eines Höhenprofils z(r, phi) ergibt, wobei gilt $F_G(r,phi) = F_L + F_S(r,phi) \Rightarrow z_G(r,phi) = z_L(r) + z(r,phi)$, wobei sich die additive Höhe z(r, phi) ausgehend von Null bis zu einem Maximalwert $z_{max}(r)$, welcher die maximale Brechkraft $F_{Smax}(r)$ liefert, nichtlinear radienabhängig ändert und sich als Funktion

$$z(r, phi) = z_{\max}(r) * w(phi)$$

mit

$$z_{\max}(r) = \sum_{j=2}^{N} c_j * r^j \qquad z_{\max}(r) = \sum_{j=1}^{N} c_j * r^{2*j}$$

oder ergibt, wobei der Radius r zwischen 0 und D/2 und der Azimutwinkel phi der Apertur zwischen 0 und $2\pi$ sich stetig ändern, wodurch die zu fertigende optische Fläche (4) mit dem spiralförmigen Höhenprofil beschrieben ist, wobei D der Linsendurchmesser ist.

6. Linse nach Anspruch 4, **dadurch gekennzeichnet, dass** die Brechkraftverteilung $F_G(r,phi)$ der Linse (1) sich aus einem Höhenprofil $z_G(r, phi)$ der zu fertigenden zweiten optischen Fläche (4) ergibt, welches sich aus der Addition des Höhenprofils $z_L(r)$ einer berechneten Basisfläche (3) und eines Höhenprofils $z(r,phi)$ ergibt, wobei gilt $F_G(r, phi) = F_L + F_S(r,phi) => z_G(r,phi) = z_L(r) + z(r, phi)$, wobei sich die additive Höhe $z(r, phi)$ ausgehend von Null bis zu einem Maximalwert $z_{max}(r,phi)$, welcher die maximale Brechkraft $F_{Smax}(r,phi)$ liefert, nichtlinear radienabhängig und vom Azimutwinkel phi der Apertur abhängig ändert und sich als Funktion

$$z(r, phi) = z_{\max}(r, phi) * w(phi)$$

mit

$$z_{\max}(r, phi) = \sum_{j=2}^{N} c_j(phi) * r^j \qquad z_{\max}(r, phi) = \sum_{j=1}^{N} c_j(phi) * r^{2*j}$$

oder ergibt, wobei der Radius r zwischen 0 und D/2 und der Azimutwinkel phi der Apertur zwischen 0 und $2\pi$ sich stetig ändern, wodurch die zu fertigende optische Fläche (4) mit dem spiralförmigen Höhenprofil beschrieben ist, wobei D der Linsendurchmesser ist.

7. Linse nach Anspruch 3, **dadurch gekennzeichnet, dass** der Brechkraftanteil mit dem spiralförmigen Verlauf $F_S(r,phi)$ sich aus der Wirkung eines optischen Gitters ergibt, welches auf eine gefertigte zweite optische Fläche (4) mit der Brechkraft $F_L$ aufgebracht ist, wobei gilt $F_G(r,phi) = F_L + F_S(r, phi)$, und sich die Frequenz des optischen Gitters von einem Grundwert Null ausgehend bis zu einem Maximalwert $Phase_{max}$, welche die maximale Brechkraft $F_{Smax}$ liefert, nichtlinear radienabhängig ändert, wobei für den spiralförmigen Brechkraftverlauf gilt

$$F_S(r, phi) = F_{S\,max}(r) * w(phi) => Phase(r, phi) = Phase_{max}(r) * w(phi)$$

$$Phase_{\max}(r) = \sum_{j=2}^{N} k_j * r^j \qquad Phase_{\max}(r) = \sum_{j=1}^{N} k_j * r^{2*j},$$

oder wobei der Radius r zwischen 0 und D/2 und der Azimutwinkel phi der Apertur zwischen 0 und $2\pi$ sich stetig ändern, wodurch das optische Gitter einen spiralförmigen Phasenverlauf aufweist, wobei D der Linsendurchmesser ist.

8. Linse nach Anspruch 4, **dadurch gekennzeichnet, dass** der Brechkraftanteil mit dem spiralförmigen Verlauf $F_S(r,phi)$ sich aus der Wirkung eines optischen Gitters ergibt, welches auf eine gefertigte zweite optische Fläche (4) mit der Brechkraft $F_L$ aufgebracht ist, wobei gilt $F_G(r,phi) = F_L + F_S(r, phi)$, und sich die Frequenz des optischen Gitters von einem Grundwert Null ausgehend bis zu einem Maximalwert $Phase_{max}$, welche die maximale Brechkraft $F_{Smax}(r,phi)$ liefert, nichtlinear radienabhängig und vom Azimutwinkel phi der Apertur abhängig ändert, wobei für den spiralförmigen Brechkraftverlauf gilt

$$F_S(r, phi) = F_{S\,max}(r, phi) * w(phi) =>$$

$$Phase(r, phi) = Phase_{max}(r, phi) * w(phi)$$

mit

$$Phase_{max}(r, phi) = \sum_{j=2}^{N} k_j(phi) * r^j \qquad Phase_{max}(r, phi) = \sum_{j=1}^{N} k_j(phi) * r^{2*j},$$

oder

wobei der Radius r zwischen 0 und D/2 und der Azimutwinkel phi der Apertur zwischen 0 und $2\pi$ sich stetig ändern, wodurch das optische Gitter einen spiralförmigen Phasenverlauf aufweist, wobei D der Linsendurchmesser ist.

9. Linse nach Anspruch 3, **dadurch gekennzeichnet, dass** der Brechkraftanteil mit dem spiralförmigen Verlauf Fs sich durch eine additive oder subtraktive Brechungsindexverteilung $\Delta n(r,phi)$ ergibt, wobei das Material der Linse (1) eine Brechungsindexverteilung aufweist, die sich von einem Grundwert $n_2$ ausgehend bis zu einem Maximalwert $\Delta n_{max}$ nichtlinear radienabhängig ändert, wobei gilt $F_G(r, phi) = F_L + F_S(r, phi)$, und für den spiralförmigen Brech-

$$\Delta n_{max}(r) = \sum_{j=2}^{N} c_j * r^j$$

kraftverlauf gilt $F_S(r,phi) = F_{Smax}(r) * w(phi) => \Delta n(r,phi) = \Delta n_{max}(r) * w(phi)$ mit

oder

$$\Delta n_{max}(r) = \sum_{j=1}^{N} c_j * r^{2*j},$$

wobei der Radius r zwischen 0 und D/2 und der Azimutwinkel phi der Apertur zwischen 0 und $2\pi$ sich stetig ändern, wodurch eine spiralförmige Brechungsindexverteilung des Linsenmaterials beschrieben ist, wobei D der Linsendurchmesser ist.

10. Linse nach Anspruch 4, **dadurch gekennzeichnet, dass** der Brechkraftanteil mit dem spiralförmigen Verlauf $F_S$ sich durch eine additive oder subtraktive Brechungsindexverteilung $\Delta n(r,phi)$ ergibt, wobei das Material der Linse (1) eine Brechungsindexverteilung aufweist, die sich von einem Grundwert $n_2$ ausgehend bis zu einem Maximalwert $\Delta n_{max}$ nichtlinear radienabhängig und vom Azimutwinkel phi der Apertur abhängig ändert, wobei gilt $F_G(r,phi) = F_L + F_S(r,phi)$, und für den spiralförmigen Brechkraftverlauf gilt $F_S(r,phi) = F_{S\,max}(r,phi) * w(phi) => \Delta n(r,phi) = \Delta n_{max}(r,phi)$

$$\Delta n_{max}(r, phi) = \sum_{j=2}^{N} c_j(phi) * r^j \qquad \Delta n_{max}(r, phi) = \sum_{j=1}^{N} c_j(phi) * r^{2*j},$$

$* w(phi)$ mit

oder

wobei

der Radius r zwischen 0 und D/2 und der Azimutwinkel phi der Apertur zwischen 0 und $2\pi$ sich stetig ändern, wodurch eine spiralförmige Brechungsindexverteilung des Linsenmaterials beschrieben ist, wobei D der Linsendurchmesser ist.

11. Verfahren zur Herstellung einer Linse (1) mit einem erweiterten Fokusbereich gemäß Anspruch 1 oder 2 mit einer Grundbrechkraft $F_L$ und einem spiralförmigen Brechkraftanteil $F_S$, welches folgende Schritte umfasst:

Schritt 1: Berechnung eines monofokalen Basissystems mit der Grundbrechkraft $F_L$ unter Festlegung der Parameter einer ersten optischen Fläche (2), der Parameter einer optischen Basisfläche (3) und einer Linsendicke d sowie einer Werkstoffart mit einem Brechungsindex und einer Abbe-Zahl;
Schritt 2: Aufaddieren oder Subtrahieren des zusätzlichen Brechkraftanteils $F_S(r,phi)$, welcher sich bezogen auf eine zur optischen Achse senkrecht stehende Ebene als Funktion der radialen Höhe r und des Azimutwinkels phi der Apertur für Werte vom Azimutwinkel phi von Null bis $2\pi$ zwischen einem Grundwert und einem Maximalwert $F_{Smax}(r)$ ungleich Null nichtlinear radienabhängig ändert, womit der zusätzliche Brechkraftanteil $F_S(r,phi)$ spiralförmig veränderlich über die Basisfläche verteilt wird,
Schritt 3: Herstellung des spiralförmigen Brechkraftanteils an der Linse und/oder auf der Linse und/oder innerhalb der Linse.

12. Verfahren nach Anspruch 11, bei dem die zusätzliche Brechkraftverteilung Fs(r,phi), welche sich zwischen dem Grundwert und dem Maximalwert ändert, durch eine Addition eines spiralförmigen Höhenprofils z(r,phi) auf eine berechnete Basisfläche (3) des Basissystems erzeugt wird, wobei die additive Höhe z eine Funktion des Radius r und des Azimutwinkels phi der Apertur ist, und wobei sich die additive Höhe z sich zwischen dem Wert Null und einem Maximalwert $z_{max}(r)$ oder $z_{max}(r,phi)$ nichtlinear radienabhängig ändert und somit ein spiralförmiges Höhenprofil der zu fertigenden zweiten optischen Fläche (4) festlegt.

13. Verfahren nach Anspruch 11, bei dem der zusätzliche Brechkraftanteil $F_S(r,phi)$, welcher sich zwischen dem Grundwert und dem Maximalwert ändert, durch eine Addition der Wirkung eines spiralförmigen Gitterprofils Phase (r,phi) auf die gefertigte zweite optische Fläche (4) erzeugt wird, wobei die gefertigte zweite optische Fläche (4) der berechneten Basisfläche (3) entspricht und das Gitterprofil eine Funktion des Radius r und des Azimutwinkels phi der Apertur ist, und sich die Brechungswirkung zwischen dem Wert Null und dem Maximalwert $Phase_{max}(r)$ oder $Phase_{max}(r,phi)$ nichtlinear radienabhängig ändert und somit das spiralförmige optische Gitter auf der gefertigten optischen Fläche (4) aufgebracht wird.

14. Verfahren nach einem der Ansprüche von 11 bis 13, bei dem der spiralförmige Brechkraftanteil $F_S(r,phi)$ an und/oder in eine phake Intraokularlinse oder eine aphake Intraokularlinse aufgeprägt und/oder eingeprägt wird.

15. Objektiv mit einem erweiterten Fokusbereich, **dadurch gekennzeichnet, dass** im Strahlengang des Objektivs eine Linse (1) mit einem erweiterten Fokusbereich gemäß der Merkmale in einem oder mehreren der Ansprüche von 1 bis 10 als abbildendes Element angeordnet ist.

**Claims**

1. Lens with an extended range of focus, wherein the lens (1) consists of a solid, transparent material and has two manufactured optical surfaces (2, 4), wherein the lens (1) has a focal power distribution $F_G$, **characterized in that** the focal power distribution $F_G$ of the lens (1), in relation to a plane perpendicular to the optical axis (10), changes as a function of the radial height r and of the azimuth angle phi of the aperture between a base value of the focal power $F_L$ not equal to zero and a maximum value $F_{Smax}$ not equal to zero and hence results in the focal power distribution $F_G(r, phi) = F_L + F_S(r, phi)$, with a spiral focal power component

$$F_S(r, phi) = F_{Smax}(r) * w(phi),$$

where $F_{Smax}(r)$ depends nonlinearly on the radius and w(phi) is a factor for the azimuthal focal power component with the spiral profile, which is described by the formula $$w(phi) = \sum_{i=1}^{M} I_i \exp\left[-a_i\left(phi - w_i\right)^2\right]$$ for the addition of focal power at the azimuth angle phi, which takes values from zero to $2\pi$, and $w_i$ are the peak positions in the angular distribution function; $I_i$ are intensity values of the individual peaks with $I_i > 0$; $a_i > 0$ are damping coefficients for the respective peak positions and i is a counter and $M \geq i$ is a final value.

2. Lens with an extended range of focus, wherein the lens (1) consists of a solid, transparent material and has two manufactured optical surfaces (2, 4), wherein the lens (1) has a focal power distribution $F_G$, **characterized in that** the focal power distribution $F_G$ of the lens (1), in relation to a plane perpendicular to the optical axis (10), changes as a function of the radial height r and of the azimuth angle phi of the aperture between a base value of the focal power $F_L$ not equal to zero and a maximum value $F_{Smax}$ not equal to zero and hence results in the focal power distribution $F_G(r, phi) = F_L + F_S(r, phi)$, with a spiral focal power component

$$F_S(r, phi) = F_{Smax}(r) * w(phi),$$

where $F_{Smax}(r)$ depends nonlinearly on the radius and w(phi) is a factor for the focal power component with the spiral profile, which is described as a linear profile by the formula $$w(phi) = \frac{phi}{2\pi}$$ for values of the azimuth angle phi from zero to $2\pi$.

3. Lens according to Claim 1 or according to Claim 2, **characterized in that** the maximum focal power $F_{Smax}(r)$ depends nonlinearly on the radius and is described by the polynomial formulae $$F_{S\,max}(r) = \sum_{j=2}^{N} c_j * r^j$$ or

$$F_{S\max}(r) = \sum_{j=1}^{N} c_j * r^{2*j} \ ,$$

with the polynomial coefficients $c_j$ for a refractive focal power and

$$F_{S\max}(r) = \sum_{j=2}^{N} k_j * r^{j} \qquad F_{S\max}(r) = \sum_{j=1}^{N} k_j * r^{2*j} \ ,$$

or

with the polynomial coefficient $k_j$ for a diffractive focal power, where j is a counter and N≥j is a final value.

4. Lens according to Claim 1 or according to Claim 2, **characterized in that** the maximum focal power $F_{S\max}(r, phi)$ depends nonlinearly on the radius and is dependent on the azimuth angle phi of the aperture and is described by

$$F_{S\max}(r, phi) = \sum_{j=2}^{N} c_j(phi) * r^{j} \qquad F_{S\max}(r, phi) = \sum_{j=1}^{N} c_j(phi) * r^{2*j} \ ,$$

the polynomial formulae

or

with the

$$F_{S\max}(r, phi) = \sum_{j=2}^{N} k_j(phi) * r^{j}$$

polynomial coefficients $c_j$ for a refractive focal power and

or

$$F_{S\max}(r, phi) = \sum_{j=1}^{N} k_j(phi) * r^{2*j} \ ,$$

with the polynomial coefficient $k_j$ for a diffractive focal power, where j is a counter and N≥j is a final value.

5. Lens according to Claim 3, **characterized in that** the focal power distribution $F_G(r, phi)$ of the lens (1) emerges from a height profile $Z_G(r, phi)$ of a second optical surface (4) to be manufactured, which emerges from adding the height profile $Z_L(r)$ of a calculated base surface (3) and a height profile z(r, phi), where

$$F_G(r, phi) = F_L + F_S(r, phi)$$
$$\Rightarrow z_G(r, phi) = z_L(r) + z(r, phi)$$

applies, where the additive height z(r, phi) changes nonlinearly dependent on the radius, starting from zero to a maximum value $z_{\max}(r)$ which supplies the maximum focal power $F_{S\max}(r)$, and emerges as a function

$$z(r, phi) = z_{max}(r) * w(phi),$$

$$z_{\max}(r) = \sum_{j=2}^{N} c_j * r^{j} \qquad z_{\max}(r) = \sum_{j=1}^{N} c_j * r^{2*j} \ ,$$

with

or

where the radius r changes continuously between 0 and D/2 and the azimuth angle phi of the aperture changes continuously between 0 and $2\pi$, as a result of which the optical surface (4) to be manufactured is described by the spiral height profile, where D is the lens diameter.

6. Lens according to Claim 4, **characterized in that** the focal power distribution $F_G(r, phi)$ of the lens (1) emerges from a height profile $z_G(r, phi)$ of the second optical surface (4) to be manufactured, which emerges from adding the height profile $z_L(r)$ of a calculated base surface (3) and a height profile z(r, phi), where

$$F_G(r, phi) = F_L + F_S(r, phi)$$
$$\Rightarrow z_G(r, phi) = z_L(r) + z(r, phi)$$

applies, where the additive height z(r, phi) changes nonlinearly dependent on the radius and dependent on the azimuth angle phi of the aperture, starting from zero to a maximum value $z_{\max}(r, phi)$ which supplies the maximum focal power $F_{S\max}(r, phi)$, and emerges as a function

$$z(r, phi) = z_{max}(r, phi) * w(phi),$$

$$z_{max}(r, phi) = \sum_{j=2}^{N} c_j(phi) * r^j \qquad z_{max}(r, phi) = \sum_{j=1}^{N} c_j(phi) * r^{2*j},$$

with          or          where the radius r changes continuously between 0 and D/2 and the azimuth angle phi of the aperture changes continuously between 0 and $2\pi$, as a result of which the optical surface (4) to be manufactured is described by the spiral height profile, where D is the lens diameter.

**7.** Lens according to Claim 3, **characterized in that** the focal power component with the spiral profile $F_S$(r, phi) emerges from the effect of an optical grating applied to a manufactured second optical surface (4) with the focal power $F_L$, where

$$F_G(r, phi) = F_L + F_S(r, phi)$$

applies, and the frequency of the optical grating changes nonlinearly dependent on the radius, starting from a base value zero to a maximum value $Phase_{max}$ which supplies the maximum focal power $F_{Smax}$, wherein the following applies to the spiral focal power profile:

$$F_S(r.phi) = F_{Smax}(r) * w(phi)$$
$$=> Phase(r, phi) = Phase_{max}(r) * w(phi),$$

with

$$Phase_{max}(r) = \sum_{j=2}^{N} k_j * r^j \quad \text{or} \quad Phase_{max}(r) = \sum_{j=1}^{N} k_j * r^{2*j},$$

where the radius r changes continuously between 0 and D/2 and the azimuth angle phi of the aperture changes continuously between 0 and $2\pi$, as a result of which the optical grating has a spiral phase profile, where D is the lens diameter.

**8.** Lens according to Claim 4, **characterized in that** the focal power component with the spiral profile $F_S$(r, phi) emerges from the effect of an optical grating applied to a manufactured second optical surface (4) with the focal power $F_L$, where

$$F_G(r, phi) = F_L + F_S(r, phi)$$

applies, and the frequency of the optical grating changes nonlinearly dependent on the radius and dependent on the azimuth angle phi of the aperture, starting from a base value zero to a maximum value $Phase_{max}$ which supplies the maximum focal power $F_{Smax}$(r, phi), wherein the following applies to the spiral focal power profile:

$$F_S(r, phi) = F_{Smax}(r, phi) * w(phi)$$
$$=> Phase(r, phi) = Phase_{max}(r, phi) * w(phi),$$

with

$$Phase_{max}(r, phi) = \sum_{j=2}^{N} k_j(phi) * r^j$$

or

**23**

$$Phase_{\max}\left(r, phi\right) = \sum_{j=1}^{N} k_j\left(phi\right) * r^{2*j} \ ,$$

where the radius r changes continuously between 0 and D/2 and the azimuth angle phi of the aperture changes continuously between 0 and $2\pi$, as a result of which the optical grating has a spiral phase profile, where D is the lens diameter.

9. Lens according to Claim 3, **characterized in that** the focal power component with the spiral profile $F_S$ emerges from an additive or subtractive refractive index distribution $\Delta n(r, phi)$, wherein the material of the lens (1) has a refractive index distribution which changes nonlinearly dependent on the radius, starting from a base value $n_2$ to a maximum value $\Delta n_{max}$, where

$$F_G(r, \ phi) \ = \ F_L \ + \ F_S(r, \ phi)$$

applies and the following applies to the spiral focal power profile:

$$F_S(r, \ phi) \ = \ F_{Smax}(r) * w(phi)$$
$$=> \ \Delta n(r, \ phi) \ = \ \Delta n_{max}(r) * w(phi),$$

with

$$\Delta n_{\max}\left(r\right) = \sum_{j=2}^{N} c_j * r^j \quad \text{or} \quad \Delta n_{\max}\left(r\right) = \sum_{j=1}^{N} c_j * r^{2*j} \ ,$$

where the radius r changes continuously between 0 and D/2 and the azimuth angle phi of the aperture changes continuously between 0 and $2\pi$, as a result of which a spiral refractive index distribution of the lens material is described, where D is the lens diameter.

10. Lens according to Claim 4, **characterized in that** the focal power component with the spiral profile $F_S$ emerges from an additive or subtractive refractive index distribution $\Delta n(r, phi)$, wherein the material of the lens (1) has a refractive index distribution, which changes nonlinearly dependent on the radius and dependent on the azimuth angle phi of the aperture, starting from a base value $n_2$ to a maximum value $\Delta n_{max}$, where

$$F_G(r, \ phi) \ = \ F_L \ + \ F_S(r, \ phi)$$

applies and the following applies to the spiral focal power profile:

$$F_S(r, \ phi) \ = \ F_{Smax}(r, \ phi) * w(phi)$$
$$=> \ \Delta n(r, \ phi) \ = \ \Delta n_{max}(r, \ phi) * w(phi),$$

with

$$\Delta n_{\max}\left(r, phi\right) = \sum_{j=2}^{N} c_j\left(phi\right) * r^j \quad \text{or} \quad \Delta n_{\max}\left(r, phi\right) = \sum_{j=1}^{N} c_j\left(phi\right) * r^{2*j} \ ,$$

where the radius r changes continuously between 0 and D/2 and the azimuth angle phi of the aperture changes continuously between 0 and $2\pi$, as a result of which a spiral refractive index distribution of the lens material is described, where D is the lens diameter.

11. Method for producing a lens (1) with an extended range of focus according to Claim 1 or 2 with a basic focal power $F_L$ and a spiral focal power component $F_S$, comprising the following steps:

Step 1: Calculating a monofocal base system with the basic focal power $F_L$ while setting the parameters of a first optical surface (2), the parameters of an optical base surface (3) and a lens thickness d as well as a material type with a refractive index and an Abbe number;

Step 2: Adding or subtracting the additional focal power component $F_S(r, phi)$ which, in relation to a plane perpendicular to the optical axis, changes nonlinearly depending on the radius as a function of the radial height r and of the azimuth angle phi of the aperture for values of the azimuth angle phi from zero to $2\pi$ between a base value and a maximum value $F_{Smax}(r)$ not equal to zero, as a result of which the additional focal power component $F_S(r, phi)$ is distributed over the base surface in a spiral changing manner,

Step 3: Producing the spiral focal power component at the lens and/or on the lens and/or within the lens.

12. Method according to Claim 11, in which the additional focal power distribution $F_S(r, phi)$, which changes between the base value and the maximum value, is generated by adding a spiral height profile z(r, phi) to a calculated base surface (3) of the base system, wherein the additive height z is a function of the radius r and of the azimuth angle phi of the aperture, and wherein the additive height z changes nonlinearly dependent on the radius between the value zero and a maximum value $z_{max}(r)$ or $z_{max}(r, phi)$ and therefore sets a spiral height profile of the second optical surface (4) to be manufactured.

13. Method according to Claim 11, in which the additional focal power component $F_S(r, phi)$, which changes between the base value and the maximum value, is generated by adding the effect of a spiral grating profile Phase(r, phi) to the manufactured second optical surface (4), wherein the manufactured second optical surface (4) corresponds to the calculated base surface (3) and the grating profile is a function of the radius r and of the azimuth angle phi of the aperture, and the refractive effect changes nonlinearly dependent on the radius between the value zero and the maximum value $Phase_{max}(r)$ or $Phase_{max}(r, phi)$ and therefore the spiral optical grating is applied to the manufactured optical surface (4).

14. Method according to one of the Claims from 11 to 13, in which the spiral focal power component $F_S(r, phi)$ is impressed upon and/or impressed into a phakic intraocular lens or an aphakic intraocular lens.

15. Lens system with an extended range of focus, **characterized in that** a lens (1) with an extended range of focus as per the features in one or more of Claims 1 to 10 is arranged in the beam path of the lens system as an imaging element.

## Revendications

1. Lentille à distance focale étendue, la lentille (1) comprenant un matériau solide et transparent et comportant deux surfaces optiques réalisées (2, 4), la lentille (1) présentant une distribution de pouvoir réfringent $F_G$, **caractérisée en ce que** la distribution de pouvoir réfringent $F_G$ de la lentille (1), rapportée à un plan perpendiculaire à l'axe optique (10), varie en fonction de la hauteur radiale r et de l'angle azimutal phi de l'ouverture entre une valeur de base du pouvoir réfringent $F_L$ différente de zéro et une valeur maximale $F_{Smax}$ différente de zéro, et la distribution de pouvoir réfringent $F_G$ est donc obtenue à partir de $F_G(r,phi) = F_L + F_S(r,phi)$, avec une proportion du pouvoir réfringent en forme de spirale $F_S(r, phi) = F_{Smax}(r)*w(phi)$, où $F_{Smax}(r)$ est dépendant non linéairement du rayon et $w(phi)$ est un facteur relatif à la proportion de pouvoir réfringent azimutale ayant l'allure en spirale, qui est déterminé par la formule

$$w(phi) = \sum_{i=1}^{M} I_i exp[-a_i(phi - w_i)^2]$$

pour l'addition du pouvoir réfringent dans l'angle azimutal phi, qui prend des valeurs de zéro à $2\pi$, et $w_i$ étant les positions de crête de la fonction de distribution d'angle ; $I_i$ étant des valeurs d'intensité des crêtes individuelles avec $I_i > 0$ ; $a_i > 0$ étant des coefficients d'amortissement pour les positions de crête respectives et i étant une valeur numérique et $M \geq i$ étant une valeur finale.

2. Lentille à distance focale étendue, la lentille (1) comprenant un matériau solide et transparent et comportant deux surfaces optiques (2, 4) réalisées, la lentille (1) comportant une distribution de pouvoir réfringent $F_G$, **caractérisée en ce que** la distribution de pouvoir réfringent $F_G$ de la lentille (1), rapportée à un plan perpendiculaire à l'axe optique (10), varie en fonction de la hauteur radiale r et de l'angle azimutal phi de l'ouverture entre une valeur de base du pouvoir réfringent $F_L$ différente de zéro et une valeur maximale $F_{Smax}$ différente de zéro et la distribution de pouvoir réfringent $F_G$ est donc obtenue à partir de $F_G(r, phi) = F_L + F_S (r,phi)$, avec une proportion du pouvoir réfringent en forme de spirale $F_S (r, phi) = F_{Smax} (r)*w(phi)$, où $F_{Smax}(r)$ est dépendant non linéairement du rayon et w(phi) est un facteur relatif à la proportion de pouvoir réfringent ayant l'allure en spirale, qui est déterminé par la formule

$$w(phi) = \frac{phi}{2\pi}$$

ayant une allure linéaire pour des valeurs de l'angle azimutal phi allant de zéro à $2\pi$.

**3.** Lentille selon la revendication 1 ou la revendication 2, **caractérisée en ce que** le pouvoir réfringent maximal $F_{Smax}(r)$

$$F_{S\max}(r) = \sum_{j=2}^{N} c_j * r^j$$ ou

dépend non linéairement du rayon et est déterminé par les formules polynomiales

$$F_{S\max}(r) = \sum_{j=1}^{N} c_j * r^{2*j}$$

avec les coefficients polynomiaux $c_j$ relatifs à un pouvoir réfringent réfractif et

$$F_{S\max}(r) = \sum_{j=2}^{N} k_j * r^j \qquad F_{S\max}(r) = \sum_{j=1}^{N} k_j * r^{2*j}$$

ou

avec le coefficient polynomial $k_j$ relatif à un pouvoir réfringent diffractif, où j est une valeur numérique et N $\geq$ j est une valeur finale.

**4.** Lentille selon la revendication 1 ou la revendication 2, **caractérisée en ce que** le pouvoir réfringent maximal $F_{Smax}(r,phi)$ dépend non linéairement du rayon et de l'angle azimutal phi de l'ouverture et est déterminé par les

$$F_{S\max}(r, phi) = \sum_{j=2}^{N} c_j(phi) * r^j \qquad F_{S\max}(r, phi) = \sum_{j=1}^{N} c_j(phi) * r^{2*j}$$

formules polynomiales ou avec les

$$F_{S\max}(r, phi) = \sum_{j=2}^{N} k_j(phi) * r^j$$

coefficients polynomiaux $c_j$ relatifs à un pouvoir réfringent réfractif et ou

$$F_{S\max}(r, phi) = \sum_{j=1}^{N} k_j(phi) * r^{2*j}$$

avec le coefficient polynomial $k_j$ relatif à un pouvoir réfringent diffractif, où j est une valeur numérique et N $\geq$ j est une valeur finale.

**5.** Lentille selon la revendication 3, **caractérisée en ce que** la distribution de pouvoir réfringent $F_G(r,phi)$ de la lentille (1) résulte d'un profil de hauteur $z_G(r, phi)$ d'une deuxième surface optique (4) à réaliser qui est obtenue à partir de l'addition du profil de hauteur $z_L(r)$ d'une surface de base (3) calculée et d'un profil de hauteur z(r, phi), où
$F_G(r,phi) = F_L + F_S (r,phi)$
=> $z_G(r,phi) = z_L(r) + z(r,phi)$, où la hauteur additive z(r,phi) varie de manière non linéaire en fonction du rayon de zéro jusqu'à une valeur maximale $z_{max}(r)$, qui donne le pouvoir réfringent maximal $F_{Smax}(r)$, et est obtenue sous la forme d'une fonction

$$z(r,phi) = z_{max}(r) *w(phi)$$

avec

$$z_{\max}(r) = \sum_{j=2}^{N} c_j * r^j \qquad z_{\max}(r) = \sum_{j=1}^{N} c_j * r^{2*j}$$

ou où le rayon r varie de manière continue entre 0 et D/2 et l'angle azimutal phi de l'ouverture varie de manière continue entre 0 et $2\pi$, de sorte que la surface optique (4) à réaliser soit décrite avec le profil de hauteur en spirale, où D est le diamètre de la lentille.

**6.** Lentille selon la revendication 4, **caractérisée en ce que** la distribution de pouvoir réfringent $F_G$(r,phi) de la lentille (1) est obtenue à partir d'un profil en hauteur $z_G$(r,phi) de la deuxième surface optique (4) à réaliser, lequel résulte de l'addition du profil de hauteur $z_L$(r) d'une surface de base (3) calculée et d'un profil de hauteur z(r,phi), où *$F_G$(r,phi) = $F_L$ + $F_S$(r,phi) => $z_G$(r,phi) = $z_L$(r) + z(r,phi),* où la hauteur additive z(r,phi) varie de manière non linéaire de zéro jusqu'à une valeur maximale $z_{max}$(r,phi), qui donne le pouvoir réfringent maximal $F_{Smax}$(r,phi), en fonction du rayon et en fonction de l'angle azimutal phi de l'ouverture et qui est obtenue sous la forme d'une fonction

$$z(r,phi) = z_{max}(r,phi)*w(phi)$$

avec

$$z_{max}(r,phi) = \sum_{j=2}^{N} c_j(phi)*r^j \qquad z_{max}(r,phi) = \sum_{j=1}^{N} c_j(phi)*r^{2*j}$$

ou                                                                                          où le rayon r varie de manière continue entre 0 et D/2 et l'angle azimutal phi de l'ouverture varie de manière continue entre 0 et $2\pi$, de sorte que la surface optique (4) à réaliser soit décrite avec le profil de hauteur en spirale, où D est le diamètre de la lentille.

**7.** Lentille selon la revendication 3, **caractérisée en ce que** la proportion de pouvoir réfringent ayant l'allure en spirale $F_S$(r,phi) résulte de l'effet d'un réseau optique qui est appliqué sur une deuxième surface optique (4) réalisée ayant le pouvoir réfringent $F_L$, avec *$F_G$(r,phi) = $F_L$ + $F_S$(r,phi)* et la fréquence du réseau optique varie de manière non linéaire en fonction du rayon depuis une valeur de base de zéro à une valeur maximale Phase$_{max}$, qui donne le pouvoir réfringent maximal $F_{Smax}$, avec pour l'allure en spirale du pouvoir réfringent

$$F_S(r,phi) = F_{Smax}(r)*w(phi)$$
$$=> Phase(r,phi) = Phase_{max}(r)*w(phi)$$

avec

$$Phase_{max}(r) = \sum_{j=2}^{N} k_j * r^j \qquad Phase_{max}(r) = \sum_{j=1}^{N} k_j * r^{2*j}$$

ou                                                                                          où le rayon r varie de manière continue entre 0 et D/2 et l'angle azimutal phi de l'ouverture varie de manière continue entre 0 et $2\pi$, de sorte que le réseau optique ait une allure de phase en spirale, où D est le diamètre de la lentille.

**8.** Lentille selon la revendication 4, **caractérisée en ce que** la proportion de pouvoir réfringent ayant l'allure en spirale $F_S$(r,phi) résulte de l'effet d'un réseau optique qui est appliqué sur une deuxième surface optique (4) réalisée ayant le pouvoir réfringent $F_L$, où $F_G$(r,phi) = $F_L$ + $F_S$(r,phi), et la fréquence du réseau optique varie de manière non linéaire en fonction du rayon depuis une valeur de base de zéro à une valeur maximale Phase$_{max}$, qui donne le pouvoir réfringent maximal $F_{Smax}$ (r,phi), en fonction de l'angle azimutal, avec pour l'allure en spirale du pouvoir réfringent

$$F_S(r,phi) = F_{Smax}(r,phi)*w(phi)$$
$$=> Phase(r,phi) = Phase_{max}(r,phi)*w(phi)$$

$$Phase_{max}(r,phi) = \sum_{j=2}^{N} k_j(phi)*r^j \qquad Phase_{max}(r,phi) = \sum_{j=1}^{N} k_j(phi)*r^{2*j}$$

avec                                                                 ou                                                                 où le rayon r varie de manière continue entre 0 et D/2 et l'angle azimutal phi de l'ouverture varie de manière continue entre 0 et $2\pi$, de sorte que le réseau optique ait une allure de phase en spirale, où D est le diamètre de la lentille.

**9.** Lentille selon la revendication 3, **caractérisée en ce que** la proportion de pouvoir réfringent en forme de spirale $F_S$ résulte d'une distribution d'indice de réfraction additive ou soustractive $\Delta$n(r,phi), le matériau de la lentille (1) présentant une distribution d'indice de réfraction qui varie de manière non linéaire en fonction du rayon depuis une valeur de base $n_2$ jusqu'à une valeur maximale $\Delta n_{max}$, avec *$F_G$(r,phi) = $F_L$ + $F_S$ (r,phi),* et avec pour l'allure du pouvoir réfringent en forme de spirale

$$F_S(r,phi) = F_{Smax}(r)*w(phi) \Rightarrow \Delta n(r,phi) = \Delta n_{max}(r)*w(phi)$$

avec

$$\Delta n_{max}(r) = \sum_{j=2}^{N} c_j * r^j \qquad \Delta n_{max}(r) = \sum_{j=1}^{N} c_j * r^{2*j}$$

ou où le rayon r varie de manière continue entre 0 et D/2 et l'angle azimutal phi de l'ouverture varie de manière continue entre 0 et $2\pi$, de sorte que l'on ait une distribution d'indice de réfraction en forme de spirale du matériau de lentille, où D est le diamètre de la lentille.

10. Lentille selon la revendication 4, **caractérisée en ce que** la proportion de pouvoir réfringent en forme de spirale $F_S$ résulte d'une distribution d'indice de réfraction additive ou soustractive $\Delta n(r,phi)$, le matériau de la lentille (1) présentant une distribution d'indice de réfraction qui varie de manière non linéaire en fonction du rayon depuis une valeur de base $n_2$ jusqu'à une valeur maximale $\Delta n_{max}$ et en fonction de l'angle azimutal phi de l'ouverture, avec $F_G(r,phi) = F_L + F_S(r,phi)$, et avec pour l'allure de pouvoir réfringent en forme de spirale

$$F_S(r,phi) = F_{Smax}(r,phi)*w(phi)$$
$$\Rightarrow \Delta n(r,phi) = \Delta n_{max}(r,phi)*w(phi)$$

avec

$$\Delta n_{max}(r,phi) = \sum_{j=2}^{N} c_j(phi) * r^j \qquad \Delta n_{max}(r,phi) = \sum_{j=1}^{N} c_j(phi) * r^{2*j}$$

ou où le rayon r varie de manière continue entre 0 et D/2 et l'angle azimutal phi de l'ouverture varie de manière continue entre 0 et $2\pi$, de sorte que l'on ait une distribution d'indice de réfraction en forme de spirale du matériau de lentille, où D est le diamètre de la lentille.

11. Procédé de fabrication d'une lentille (1) à distance focale étendue selon la revendication 1 ou 2 ayant un pouvoir réfringent de base $F_L$ et une proportion de pouvoir réfringent en forme de spirale $F_S$, le procédé comprenant les étapes suivantes :

Étape 1 : Calcul d'un système de base monofocale ayant le pouvoir réfringnet de base $F_L$ avec spécification des paramètres d'une première surface optique (2), des paramètres d'une surface de base optique (3) et d'une épaisseur de lentille d ainsi que d'un type de matériau ayant un indice de réfraction et un nombre d'Abbe ;
Étape 2 : ajouter ou soustraire la proportion de pouvoir réfringent supplémentaire $F_S(r,phi)$ qui varie, rapportée à un plan perpendiculaire à l'axe optique, de manière non linéaire en fonction du rayon, en fonction de la hauteur radiale r et de l'angle azimutal phi de l'ouverture pour des valeurs de l'angle azimutal phi allant de zéro à $2\pi$ entre une valeur de base et une valeur maximale $F_{Smax}(r)$ différente de zéro, la proportion de pouvoir réfringent supplémentaire $F_S(r,phi)$ étant distribuée de manière variable en forme de spirale sur la surface de base,
Étape 3 : réaliser la proportion de pouvoir réfringent au niveau de la lentille et/ou sur la lentille et/ou dans la lentille.

12. Procédé selon la revendication 11, dans lequel la distribution de pouvoir réfringent supplémentaire $F_S(r,phi)$, qui varie entre la valeur de base et la valeur maximale, est générée par addition d'un profil de hauteur en forme de spirale $z(r,phi)$ à une surface de base (3) calculée du système de base, la hauteur additive z étant une fonction du rayon r et de l'angle azimutal phi de l'ouverture, et la hauteur additive z variant non linéairement en fonction du rayon entre la valeur zéro et une valeur maximale $Z_{max}(r)$ ou $Z_{max}(r,phi)$ et fixant ainsi un profil de hauteur en forme de spirale de la deuxième surface optique (4) à réaliser.

13. Procédé selon la revendication 11, dans lequel la proportion de pouvoir réfringnet $F_S(r,phi)$, qui varie entre la valeur de base et la valeur maximale, est générée par addition de l'effet d'une profil de réseau en forme de spirale Phase$(r,phi)$ à la deuxième surface optique (4) réalisée, la deuxième surface optique (4) réalisée correspondant à la surface de base (3) calculée et le profil de réseau étant une fonction du rayon r et de l'angle azimutal phi de l'ouverture, et l'effet de réfraction variant de manière non linéaire en fonction du rayon entre la valeur zéro et la valeur maximale Phase$_{max}(r)$ ou Phase$_{max}(r,phi)$ et le réseau optique en formant de spirale étant ainsi appliqué sur la surface optique (4) réalisée.

**14.** Procédé selon l'une des revendications 11 à 13, dans lequel la proportion de pouvoir réfringent en forme de spirale $F_S(r,phi)$ est imprimée sur et/ou dans une lentille intraoculaire phaque ou une lentille intraoculaire aphaque.

**15.** Objectif à distance étendue, **caractérisé en ce qu'**une lentille (1) à distance focale étendue selon les caractéristiques d'une ou de plusieurs des revendications 1 à 10 est disposée en tant qu'élément de reproduction dans le trajet de rayons de l'objectif.

Figur 1

Figur 2

Figur 3

Figur 4

Figur 5

Figur 6

Figur 7

Figur 8

Figur 9

Figur 10

Figur 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5982543 A
- US 6120148 A
- US 6536899 B
- US 2006176572 A
- WO 10083546 A
- EP 0622653 A1
- US 20100002310 A1
- DE 102009033984 A1